# EUROPEAN PATENT APPLICATION

(11) **EP 3 974 416 A1**
(43) Date of publication of application: **30.03.2022**
(21) Application number: 20197770.9
(22) Date of filing: 23.09.2020
(51) Int. Cl.: C07D 249/04, C07D 401/12, C07D 401/14, C07D 403/12, C07D 405/12, A61P 31/04, A61K 31/4192

(54) **NOVEL ALBICIDIN DERIVATIVES, THEIR USE AND SYNTHESIS**

(71) Applicant: Technische Universität Berlin, 10623 Berlin (DE)
(72) Inventor: PROF. DR. SÜßMUTH, Roderich, 10629 Berlin (DE); DR. WESTON, John, 65779 Kelkheim (DE); BEHROZ, Iraj, 13349 Berlin (DE); KLEEBAUER, Leonardo, 10623 Berlin (DE); ZBOROVSKY, Lieby, 14052 Berlin (DE); HOMMERNICK, Kay, 10967 Berlin (DE)
(74) Representative: Maikowski & Ninnemann Patentanwälte Partnerschaft mbB

(57) **Abstract**

The present invention relates to an albidicin derivatives, in particular to amid bond isosteres compound according to general formula (1).

## Description

The present invention relates to albicidin derivatives, in particular to amide bond isostere derivatives of albicidine.

Albicidin is a natural product, isolated from *Xanthomonas albilineans* and heterologously expressed in *Xanthomonas axonopodis* pv *vesicatoria.* Its structure (see below) is based on peptides and amino acids, but it does not contain any proteinogenic amino acids.

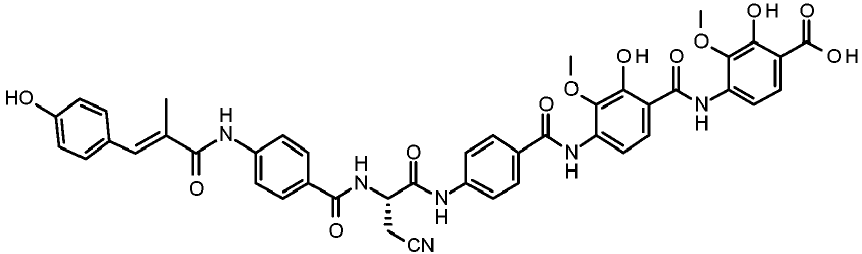

Albicidin is, on the one hand, a causative agent of the leaf scald disease in sugar cane and on the other hand a DNA-gyrase-inhibitor of prokaryotic cells (gram-positive and -negative). The mentioned properties make the natural product albicidin a potential antibiotic.

The known molecular structure of albicidin and available synthetic routes allows the development of a plurality of novel derivatives that may exhibit potential antimicrobial activities.

The problem underlying the present invention is the provision of new compounds, which comprise antibiotic properties, a method of their synthesis and their use. This problem is attained by the subject-matter of the independent claims.

### Terms and Definitions

The term "purity" as used in the context of the present specification with respect to a preparation of a certain compound refers to the content of said compound relative to the sum of all compounds contained in the preparation. The term "compound" in this context is to be understood as a compound according to the invention (or any specific embodiments thereof) as well as any salts, hydrates or solvates thereof. Thus, the respective salts, hydrates or solvates are not considered as impurities according to the previous definition. The "purity" of a compound may be determined using elemental analysis, HPLC analysis using UV diode array detection also in combination with mass spectrometry detection, or quantitative NMR analysis.

The term "substituted" refers to the addition of a substituent group to a parent moiety. "Substituent groups" can be protected or unprotected and can be added to one available site or to many available sites in a parent moiety. Substituent groups may also be further substituted with other substituent groups and may be attached directly or by a linking group such as an alkyl, an amide or hydrocarbyl group to a parent moiety. "Substituent groups" amenable herein include, without limitation, halogen, subst. oxygen, subst. nitrogen, subst. sulphur, hydroxyl, alkyl, alkenyl, alkynyl, acyl (-C(O)R^{a}), carboxyl (-C(O)OR^{a}), aliphatic groups, alicyclic groups, alkoxy, substituted oxy (-OR^{a}), aryl, aralkyl, heterocyclic radical, heteroaryl, heteroarylalkyl, amino (-N(R^{b})(R^{c})), imino(=NR^{b}), amido (-C(O)N(R^{b})(R^{c}) or -N(R^{b})C(O)R^{a}), hydrazine derivates -NR^{a}NR^{b}R^{c} , tetrazolyl (CN₄H₁), azido (-N₃), nitro (-NO₂), cyano (-CN), isocyano (-NC), cyanato (-OCN), isocyanato (-NCO), thiocyanato (-SCN); isothio-cyanato (-NCS); carbamido (-OC(O)N(R^{b})(R^{c}) or -N(R^{b})C(O)OR^{a}), substituted thio (-SR^{b}), sulfinyl (-S(O)R^{b}), sulfonyl (-S(O)₂R^{b}), sulfonamidyl (-S(O)₂N(R^{b})(R^{c}) or -N(R^{b})S(O)₂R^{b}) and fluorinated groups such as -CH2CF₃, -CHFCF₃, -CF₂CF₃, -CHF2, -CH2F, -CF₃, -OCF₃, -SCF₃, -SOCF₃ or-SO₂CF₃. Wherein each R^{a}, R^{b} and R^{c} is, independently, H or a further substituent group with a preferred list including without limitation, H, alkyl, alkenyl, alkynyl, cycloalkyl, alkoxy, acyl, aryl, heteroaryl, alicyclyl, heterocyclyl and heteroarylalkyl.

As used herein the term "alkyl," refers to a saturated straight or branched hydrocarbon moiety containing up to 8, particularly up to 4 carbon atoms. Examples of alkyl groups include, without limitation, methyl, ethyl, propyl, butyl, isopropyl, n-hexyl, octyl, and the like. Alkyl groups typically include from 1 to about 8 carbon atoms (C₁-C₈ alkyl), particularly with from 1 to about 4 carbon atoms (C₁-C₄ alkyl).

As used herein the term "cycloalkyl" refers to an interconnected alkyl group forming a saturated or unsaturated ring (whereby an unsaturated cycle can also be defined as "cycloalkenyl") or polyring structure containing 3 to 10, particularly 5 to 10 carbon atoms. Examples of cycloalkyl groups include, without limitation, cyclopropyl, cyclopentyl, cyclohexyl, norbornyl, decalinyl or adamantyl (derived from tricyclo[3.3.1.1]decane), and the like. Cycloalkyl groups typically include from 5 to 10 carbon atoms (C₅-C₁₀ cycloalkyl).

Alkyl or cycloalkyl groups as used herein may optionally include further substituent groups. A substitution on the cycloalkyl group also encompasses an aryl, a heterocyclyl or a heteroaryl substituent, which can be connected to the cycloalkyl group via one atom or two atoms of the cycloalkyl group (like tetraline).

As used herein the term "haloalkyl," refers to a saturated straight or branched hydrocarbon moiety containing 1 to 8, particularly 1 to 4, carbon atoms and at least one halogen atom, in particular Cl or F, connected to a carbon atom. Examples of haloalkyl groups include, without limitation, CF₃, CHF₂, CH₂F, CH₂CF₃, CH₂CHF₂, CH₂CH₂F, CHFCF₃, CHFCHF₂, CHFCH₂F, CF₂CF₃, CF₂CHF₂, CF₂CH₂F and the like. Haloalkyl groups typically include 1 to 4 carbon atoms (C₁-C₄ haloalkyl). More particularly haloalkyl groups comprise only F as halogen atoms.

As used herein the term "halo cycloalkyl" refers to an interconnected alkyl group forming a saturated or unsaturated ring or polyring structure containing 3 to 10, particularly 5 to 10 carbon atoms and at least one halogen atom, in particular Cl or F, connected to a carbon atom. Examples of halo cycloalkyl groups include, without limitation, fluorocyclopropyl, chlorocyclohexyl, dichlorocyclohexyl, chloroadamantyl, and the like. Halo cycloalkyl groups typically include from 5 to 10 carbon atoms (C₅-C₁₀ cycloalkyl). More particularly cyclohaloalkyl groups comprise only F as halogen atoms.

Halo alkyl or halo cycloalkyl groups as used herein may optionally include further substituent groups. A substitution on the halo cycloalkyl group also encompasses an aryl, a heterocyclyl or a heteroaryl substituent, which can be connected to the halo cycloalkyl group via one atom or two atoms of the halo cycloalkyl group (like tetraline).

As used herein the term "alkenyl," refers to a straight or branched hydrocarbon chain moiety containing up to 8 carbon atoms and having at least one carbon-carbon double bond. Examples of alkenyl groups include, without limitation, ethenyl, propenyl, butenyl, 1-methyl-2-buten-1-yl, dienyl groups such as 1,3-butadienyl and the like. Alkenyl groups typically include from 2 to about 8 carbon atoms, more typically from 2 to about 4 carbon atoms. Alkenyl groups as used herein may optionally include further substituent groups.

As used herein the term "alkynyl," refers to a straight or branched hydrocarbon moiety containing up to 8 carbon atoms and having at least one carbon-carbon triple bond. Examples of alkynyl groups include, without limitation, ethynyl, 1-propynyl, 1-butynyl, and the like. Alkynyl groups typically include from 2 to about 8 carbon atoms, more typically from 2 to about 4 carbon atoms. Alkynyl groups as used herein may optionally include further substituent groups.

As used herein the term "carboxy," refers to an carboxy (-C(=O)-O- or -O-C(=O)-) alkyl moiety containing 1 to 8, particularly 1 to 4 carbon atoms comprising at least one carboxy moiety, wherein the carboxy group is used to attach the carboxy group to a parent molecule. Examples of carboxy groups include without limitation, formate, acetate, lactate, citrate, oxalate and the like. Carboxy groups as used herein may optionally include further substituent groups. In particular "carboxy" groups include straight or branched polycarboxy groups (polyester), which comprise several interconnected monomeric carboxy groups (e. g. -C(=O)-O-CH₂-CH₂-). Non limiting examples are polyethylester or polyacrylate.

As used herein the term "alkoxy," refers to an oxygen alkyl moiety containing 1 to 8, particularly 1 to 4 carbon atoms comprising at least one oxygen moiety, wherein the oxygen atom is used to attach the alkoxy group to a parent molecule. Examples of alkoxy groups include without limitation, methoxy, ethoxy, n-propoxy, isopropoxy, n-butoxy, sec-butoxy, tert-butoxy, n-pentoxy, neopentoxy, n-hexyloxy and the like. Alkoxy groups as used herein may optionally include further substituent groups. In particular "alkoxy" groups include straight or branched polyalkoxy groups (polyether), which comprise several interconnected monomer alkoxy groups (e. g. -O-CH2-CH2-). Non limiting examples are groups derived from polyethyleneglycol (PEG) or polypropylenglycol (PPG).

As used herein the term "heterocyclyl" refers to an interconnected alkyl group forming a saturated or unsaturated ring or polyring structure containing 3 to 10, particularly 5 to 10 carbon atoms in which at least one carbon atom is replaced with an oxygen, a nitrogen or a sulphur atom forming a non-aromatic structure. Examples of heterocyclyl groups include, without limitation, oxalanyl, pyrrolidinyl or piperidinyl. Heterocyclic groups as used herein may optionally include further substituent groups. A substitution on the heterocyclic group also encompasses an aryl, a cycloalkyl or a heteroaryl substituent, which can be connected to the heterocyclic group via one atom or two atoms of the heterocyclic group (comparable to indole or indoline).

As used herein the term "aryl" refers to a hydrocarbon with alternating double and single bonds between the carbon atoms forming an aromatic ring structure, in particular a six (C₆) to ten (C₁₀) membered ring or polyring structure. The term "heteroaryl" refers to aromatic structures comprising a five to ten membered ring or polyring structure, comparable to aryl compounds, in which at least one member is an oxygen or a nitrogen or a sulphur atom. Due to simplicity reasons they are denominated C₅ to C₁₀ heteroaryl, wherein at least one carbon atom is replaced with an oxygen, a nitrogen or a sulphur atom forming an aromatic structure. For example a C₅ heteroaryl comprises a five membered ring structure with at least one carbon atom being replaced with an oxygen, a nitrogen or a sulphur atom. Examples for such a C₅ heteroaryl are triazolyl, pyrazolyl, imidazolyl, thiophenyl, furanyl or oxazolyl. A C₆ heteroaryl can be pyridyl, pyrimidinyl or triazinyl. A C₉ heteroaryl can be indolyl and a C₁₀ heteroaryl can be quinolinyl. Aryl or hetero aryl groups as used herein may optionally include further substituent groups. A substitution on the hetero aryl group also encompasses an aryl, a cycloalkyl or a heterocyclyl substituent, which can be connected to the hetero aryl via one atom or two atoms of the hetero aryl group (comparable to indole). The same applies to an aryl group.

### Description of the invention

According to the invention compounds are provided having the general formulae (1) 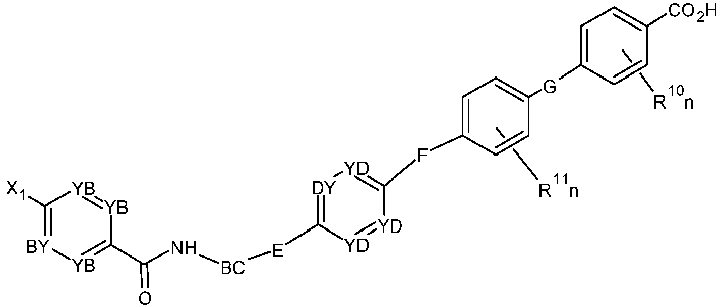
a) with X₁ in formulae being
   a substituted or unsubstituted C₁-C₁₆ alkyl, in particular a substituted or unsubstituted C₁-C₈ alkyl;
   a substituted or unsubstituted C₂-C₁₆ alkenyl, a substituted or unsubstituted C₂-C₈ alkenyl;
   a substituted or unsubstituted C₂-C₁₆ alkynyl, a substituted or unsubstituted C₂-C₈ alkynyl;
   -NHCNHRₐ;
   -(NHCN)Rₐ, wherein (NHCN) forms a ring with the benzyl of formulae (Ia);
   E-CONH-, wherein E is a substituted or unsubstituted C₅-C₁₀ heteroaryl, a substituted or unsubstituted C₆-C₁₀ aryl, or
   -CHR_{b}-ORₐ;
   with Rₐ being a substituted or unsubstituted C₆-C₁₀ aryl, a substituted or unsubstituted C₆-C₁₀ hetero aryl, and
   R_{b} being H, C₁-C₆ alkyl, preferably C₁-C₄ alkyl;
b) with at least one, preferably at least two or all of E, F, G being -CO-NH-; and/or
   with at least one of E, F and G being independently from each other selected from the group comprising 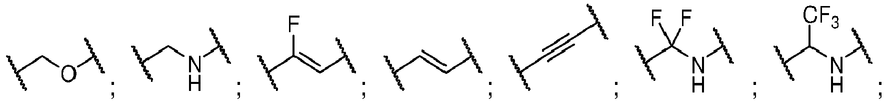 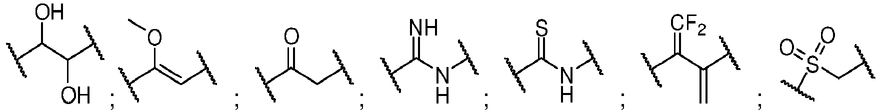 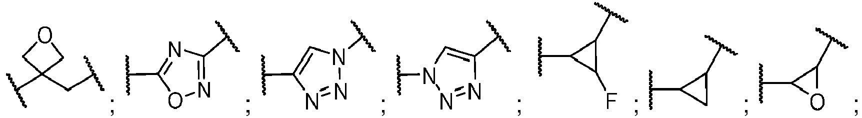 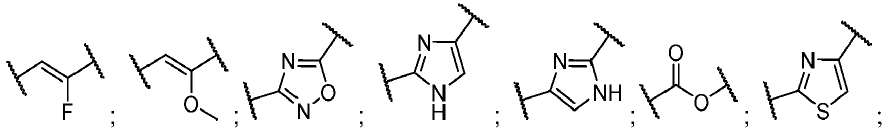 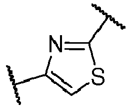 Or E, F, G are part of a ring formed with one of the adjacent phenyl rings, such as 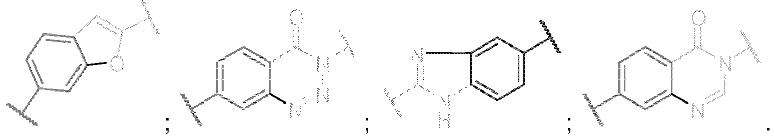
c) with BC being selected from 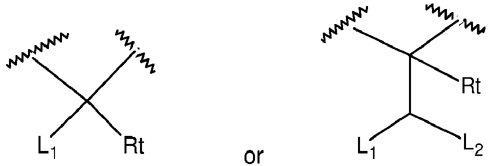
   with L₁ being a substituted or unsubstituted aromatic heterocycle or a substituted or unsubstituted non-aromatic heterocycle, or -NHR^{d} or -NR^{d}₂;
   with Rt being selected from H or C₁-C₄ alkyl,
   with L₁ and Rt forming a non-aromatic heterocycle, in particular a N-heterocyclic ring, which is optionally substituted,
   with L₂ being selected from -H, -OH, -OR^{d}, and substituted or unsubstituted -C₁-C₄ alkyl, C₁-C₆ alkoxycarbonyl and C₁-C₆ alkylaminocarbonyl,
   with R^{d} being selected from a substituted or unsubstituted C₁-C₁₆ alkyl, , a substituted or unsubstituted C₂-C₁₆ alkenyl, in particular a substituted or unsubstituted C₁-C₈ alkyl, a substituted or unsubstituted C₂-C₈ alkenyl, a substituted or unsubstituted C₃-C₁₀ cycloalkyl, and all moieties optionally substituted with F,
   or with BC being selected from 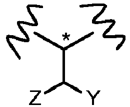
   with Y being selected from -CN, -C(=O)OH, -C(=O)OCH₃, -C(=O)OCH₂CH₃,-C(=O)NHCH₃, -C(=O)NHCH₂CH₃, -C(=O)N(CH₃)₂, -C(=O)N(CH₂CH₃)₂,-C(=O)N(CH₃)(CH₂CH₃), -CF₃ or -C(=O)NH₂, and
   with Z being selected from -H, -OH, -CH₃, -CH₂CH₃, -CCH, -OCH₃ ,-NH2, -NHCH₃, -N(CH₃)₂, -N(CH₃)₃⁺,
d) with n of R¹⁰ₙ and n of R¹¹ₙ being independently from each other 0, 1, 2, 3 or 4, in particular n of R¹⁰ₙ and n of R¹¹ₙ being 0, 1, 2 or 3, and
   with each R¹⁰ and R¹¹ being selected independently from any other R¹⁰ and R¹¹ from -OH, -F, -Cl, -Br, -I, -CCH, -CN, -OC₁-C₆ alkyl, -OCF₃, -NH₂, -NHCH₃, -N(CH₃)₂,-C₁-C₆ alkyl, -OCH₂-NH₂, -OCH₂-CO₂H, -OCH₂-CN, in particular from -OH, -F, -OCH₃,-OC₂H₅, -OiC₃H₇, -OnC₃H₇, -OCF₃, more particulary -OH, OCH₃, -OC₂H₅ or -OiPr respectively;
e) with YB being independently from each other N or CR¹⁴ and YD being independently from each other N or CR¹³;
   with each R¹³ and R¹⁴ independently being selected from -H, -OH, -F, -OCH₃,-OC₂H₅, -OC₃H₇, -OCF₃, -CF₃ or -(CH₂)ₘ-ORₐ,
   with Rₐ being selected from hydrogen, -CH₃, -CH₂CH₃, -CH₂CH₂CH₃,-CH₂CH₂CH₂CH₃, -CH(CH₃)₂, -CH₂CH(CH₃)₂, -C(CH₃)₃, -C₆H₅ -CH₂C₆H₅,
   with m being selected from 1 or 2;
   more particularly with R¹³ being -H, -OH, -OCH₃, -OC₂H₅ or -OiPr and R¹⁴ being -H or -F respectively
   wherein the following structure is disclaimed:

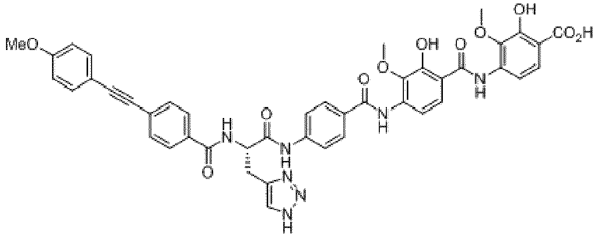

According to one embodiment, the invention relates to compounds having a molecular structure as defined by formula (1a) 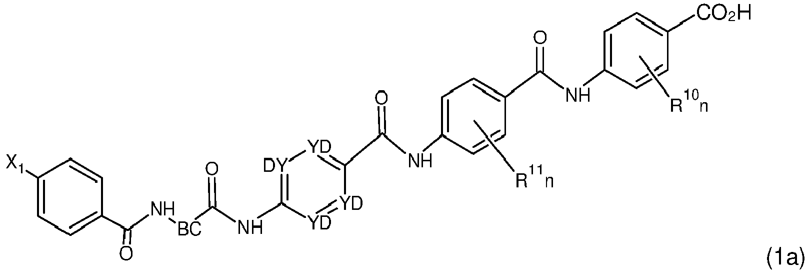
a) with X₁ in formulae (Ia) being
   a substituted or unsubstituted C₁-C₁₆ alkyl, in particular a substituted or unsubstituted C₁-C₈ alkyl;
   a substituted or unsubstituted C₂-C₁₆ alkenyl, in particular a substituted or unsubstituted C₂-C₈ alkenyl;
   a substituted or unsubstituted C₂-C₁₆ alkynyl, in particular a substituted or unsubstituted C₂-C₈ alkynyl;
   -NHCNHRₐ;
   -(NHCN)Rₐ, wherein (NHCN) forms a ring with the phenyl of formulae (Ia);
   - E-CONH-, wherein E is a substituted or unsubstituted C₅-C₁₀ heteroaryl, a substituted or unsubstituted C₆-C₁₀ aryl, or
   -CHR_{b}-ORₐ;
      with
   Rₐ being a substituted or unsubstituted C₆-C₁₀ aryl, a substituted or unsubstituted C₆-C₁₀ hetero aryl, and
   R_{b} being H, C₁-C₆ alkyl, preferably C₁-C₄ alkyl;
c) with BC being selected from 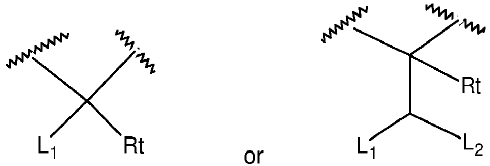
   with L₁ being a substituted or unsubstituted aromatic heterocycle or a substituted or unsubstituted non-aromatic heterocycle, or -NHR^{d} or -NR^{d}₂;
   with Rt being selected from H or C₁-C₄ alkyl,
   with L₁ and Rt forming a non-aromatic heterocycle, in particular a N-heterocyclic ring, which is optionally substituted,
   with L₂ being selected from -H, -OH, -OR^{d}, and substituted or unsubstituted -C₁-C₄ alkyl, C₁-C₆ alkoxycarbonyl and C₁-C₆ alkylaminocarbonyl,
   with R^{d} being selected from a substituted or unsubstituted C₁-C₁₆ alkyl, , a substituted or unsubstituted C₂-C₁₆ alkenyl, in particular a substituted or unsubstituted C₁-C₈ alkyl, a substituted or unsubstituted C₂-C₈ alkenyl, a substituted or unsubstituted C₃-C₁₀ cycloalkyl, and all moieties optionally substituted with F,
   or with BC being selected from 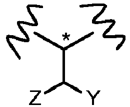
   with Y being selected from -CN, -C(=O)OH, -C(=O)OCH₃, -C(=O)OCH₂CH₃,-C(=O)NHCH₃, -C(=O)NHCH₂CH₃, -C(=O)N(CH₃)₂, -C(=O)N(CH₂CH₃)₂,-C(=O)N(CH₃)(CH₂CH₃), -CF₃ or -C(=O)NH₂, and
   with Z being selected from -H, -OH, -CH₃, -CH₂CH₃, -CCH, -OCH₃ ,-NH2, -NHCH₃, -N(CH₃)₂, -N(CH₃)₃⁺,
d) with n of R¹⁰ₙ and n of R¹¹ₙ being independently from each other 0, 1, 2, 3 or 4, in particular n of R¹⁰ₙ and n of R¹¹ₙ being 0, 1, 2 or 3, and
   with each R¹⁰ and R¹¹ being selected independently from any other R¹⁰ and R¹¹ from -OH, -F, -Cl, -Br, -I, -CCH, -CN, -OC₁-C₆ alkyl, -OCF₃, -NH₂, -NHCH₃, -N(CH₃)₂,-C₁-C₆ alkyl, -OCH2-NH2, -OCH₂-CO₂H, -OCH2-CN, in particular from -OH, -F, -OCH₃,-OC₂H₅, -OiC₃H₇, -OnC₃H₇, -OCF₃, more particular -OH, OCH₃, -OC₂H₅ or -OiPr respectively;
e) with YD being independently from each other N or CH;
wherein the following structure is disclaimed:

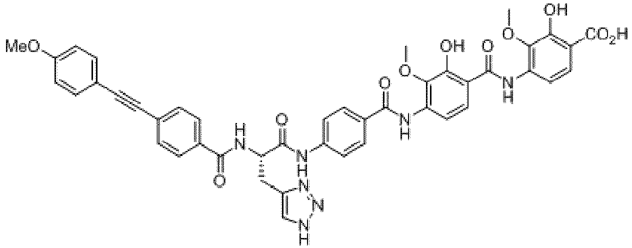

In one embodiment of the present compound according to general formulae (1a)
X₁ is -CH₂CHRa, -CHCHRa, -CCRₐ,
wherein Rₐ being a substituted or unsubstituted C₆-C₁₀ aryl, substituted or unsubstituted C₅-C₁₀ heteroaryl, in particular N-heteroaryl, wherein at least one substituent may be -OH,-OAlkyl, in particular -OCH₃, -CN, halogen, in particular F,
Rₐ being most preferably phenyl, methoxyphenyl, cyano benzyl, fluoro benzyl.

In a preferred embodiment of the present compound according to general formulae (1a) X₁ in formulae (Ia) is selected from -CC-C₆H₄-CN, - CC-C₆H₄-OH, - CC-C₆H₄-OCH₃, - CC-C₅H₃N-OCH₃, -CC-C₆H₅-F; - CHCH-C₆H₄-OH; - CH₂CH₂-C₆H₄-OH.

In another embodiment of the present compound according to general formulae (1a) X₁ in is-NHCNHRₐ or -(NHCN)Rₐ, wherein (NHCN) forms a ring with the phenyl of formulae (Ia), wherein Rₐ is phenyl.

In yet another embodiment of the present compound according to general formulae (1a) X₁ in is E-CONH-, wherein E being
a substituted or unsubstituted C₆-C₁₀ aryl, in particular substituted or unsubstituted benzyl, naphthyl, wherein the substituent may be an alkyl, alkenyl or alkynyl, such as a C₁-C₆ alkyl, a C₂-C₈ alkenyl or a C₂-C₈ alkynyl,
a substituted or unsubstituted N-heteroaryl, such as a triazoles or a tetrazole, imidazole, indole, benzimidazole, quinoline, or
   -CHR_{b}-ORₐ, wherein Rₐ is phenyl and R_{b} is H or -CH₃.

In a specific embodiment E may be a 1,2,3 - triazole, a tetrazole, an indole with at least one OH-substituent, naphthyl with at least one OH-substituent, -CHCH₃-O-C₆H₄OH, -CH₂-O-C₆H₄OH.

In a preferred embodiment the present compound may be of the general formulae (2a) 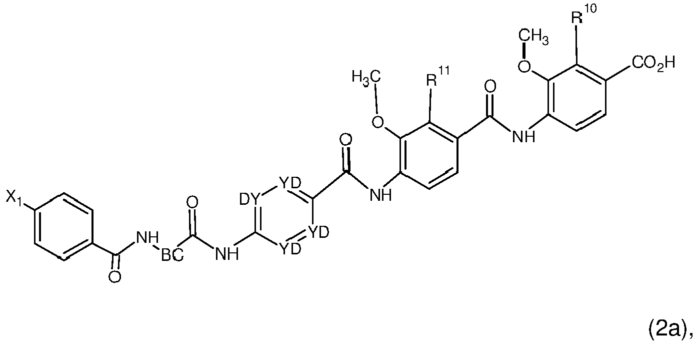 wherein X¹, BC, YD, R¹¹ and R¹⁰ have the above meaning.

In a further preferred embodiment the present compound may be of the general formulae (3a) 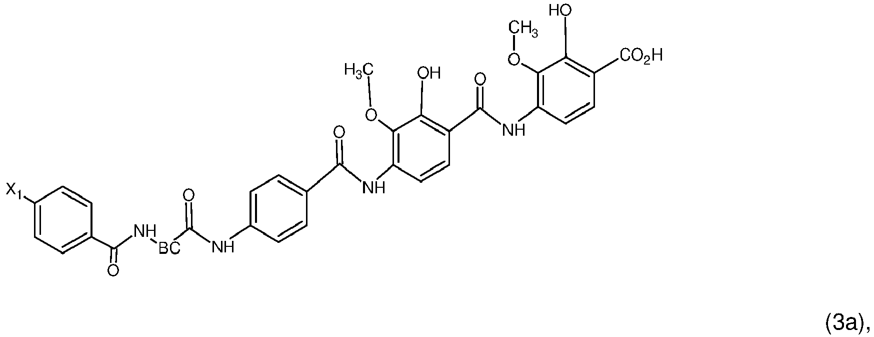 wherein X¹ and BC have the above meaning.

According to another embodiment, the invention relates to compounds having a molecular structure as defined by general formula (1b) 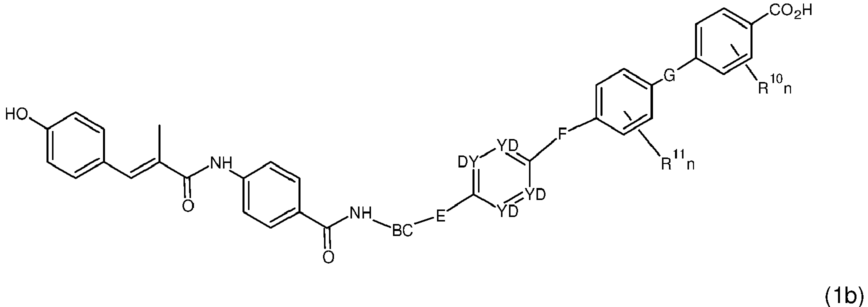 with at least one, preferably at least two of E, F, G being -CO-NH-; and
with at least one of E, F and G being independently from each other selected from the group comprising 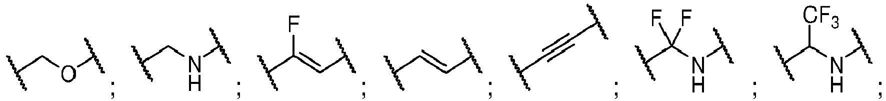 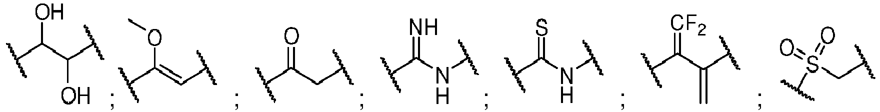 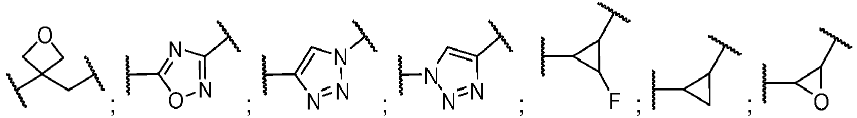 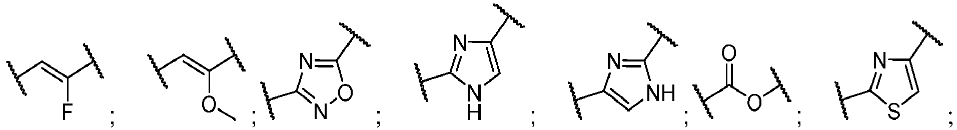 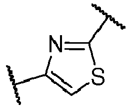 or E, F, G are part of a ring formed with one of the adjacent phenyl rings, such as 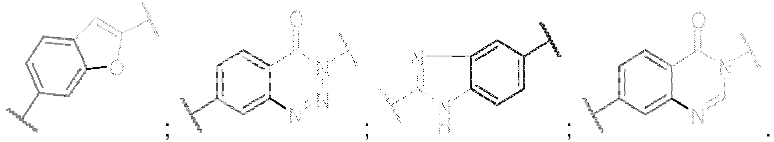
c) with BC being selected from 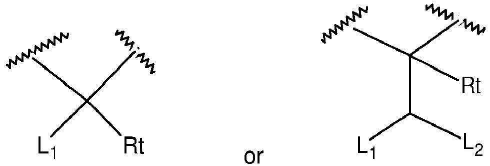
   with L₁ being a substituted or unsubstituted aromatic heterocycle or a substituted or unsubstituted non-aromatic heterocycle, or -NHR^{d} or -NR^{d}₂;
   with Rt being selected from H or C₁-C₄ alkyl,
   with L₁ and Rt forming a non-aromatic heterocycle, in particular a N-heterocyclic ring, which is optionally substituted,
   with L₂ being selected from -H, -OH, -OR^{d}, and substituted or unsubstituted -C₁-C₄ alkyl, C₁-C₆ alkoxycarbonyl and C₁-C₆ alkylaminocarbonyl,
   with R^{d} being selected from a substituted or unsubstituted C₁-C₁₆ alkyl, a substituted or unsubstituted C₂-C₁₆ alkenyl, in particular a substituted or unsubstituted C₁-C₈ alkyl, a substituted or unsubstituted C₂-C₈ alkenyl, a substituted or unsubstituted C₃-C₁₀ cycloalkyl, and all moieties optionally substituted with F,
   or with BC being selected from 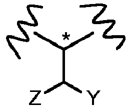
   with Y being selected from -CN, -C(=O)OH, -C(=O)OCH₃, -C(=O)OCH₂CH₃,-C(=O)NHCH₃, -C(=O)NHCH₂CH₃, -C(=O)N(CH₃)₂, -C(=O)N(CH₂CH₃)₂,-C(=O)N(CH₃)(CH₂CH₃), -CF₃ or -C(=O)NH₂, and
   with Z being selected from -H, -OH, -CH₃, -CH₂CH₃, -CCH, -OCH₃ ,-NH2, -NHCH₃, -N(CH₃)₂, -N(CH₃)₃⁺,
d) with n of R¹⁰ₙ and n of R¹¹ₙ being independently from each other 0, 1, 2, 3 or 4, in particular n of R¹⁰ₙ and n of R¹¹ₙ being 0, 1, 2 or 3, and
   with each R¹⁰ and R¹¹ being selected independently from any other R¹⁰ and R¹¹ from -OH, -F, -Cl, -Br, -I, -CCH, -CN, -OC₁-C₆ alkyl, -OCF₃, -NH₂, -NHCH₃, -N(CH₃)₂,-C₁-C₆ alkyl, -OCH2-NH2, -OCH₂-CO₂H, -OCH2-CN, in particular from -OH, -F, -OCH₃,-OC₂H₅, -OiC₃H₇, -OnC₃H₇, -OCF₃, more particularly -OH, OCH₃, -OC₂H₅ or -OiPr respectively;
e) with YD being independently from each other N or CH;

In one embodiment of the compound of general formula (1c) one of E, F, G are as follows:
E is 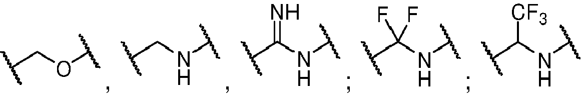 F is 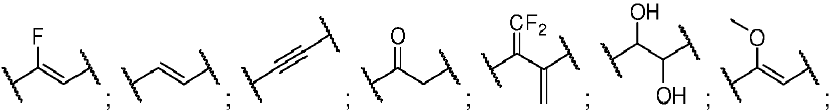 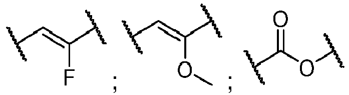
G is 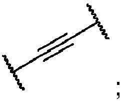
or one of E, F, G are part of a ring formed with one of the adjacent phenyl rings, such as 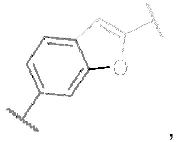 wherein at least one, preferably two of E, F or G is -CO-NH-.

In a preferred embodiment the present compound may be of the general formulae (2c) 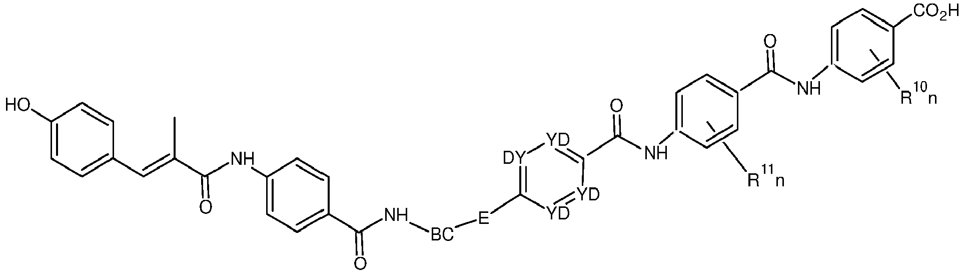 wherein BC, YD, E, R¹¹ and R¹⁰ have the above meaning.

In a preferred embodiment the present compound may be of the general formulae (3c) 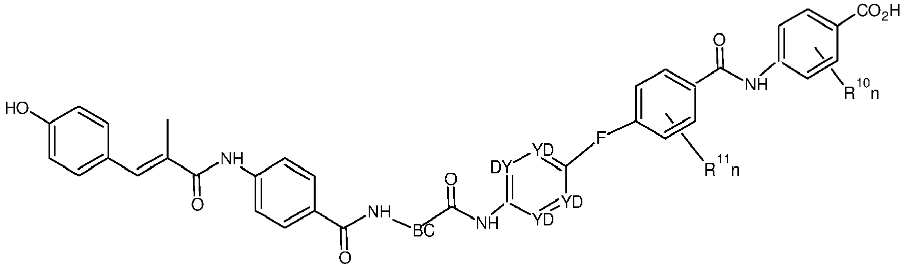 wherein BC, YD, F, R¹¹ and R¹⁰ have the above meaning.

In a preferred embodiment the present compound may be of the general formulae (4c) 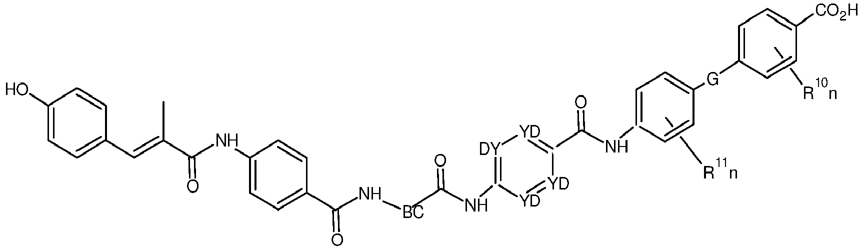 wherein BC, YD, G, R¹¹ and R¹⁰ have the above meaning.

In yet another preferred embodiment the present compound may be of the general formulae (5c) 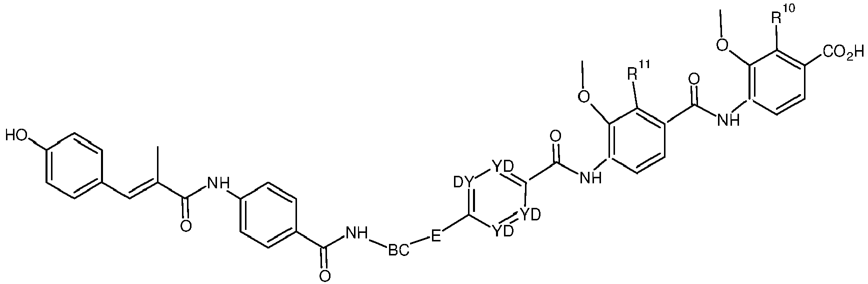 wherein BC, YD, E, R¹¹ and R¹⁰ have the above meaning.

In yet another preferred embodiment the present compound may be of the general formulae (6c) 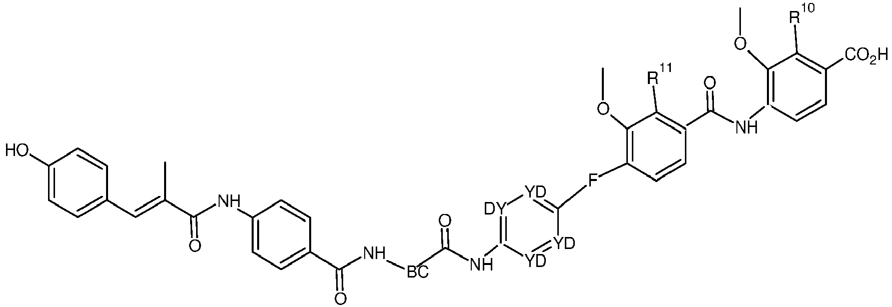 wherein BC, YD, F, R¹¹ and R¹⁰ have the above meaning.

In yet another preferred embodiment the present compound may be of the general formulae (5c) 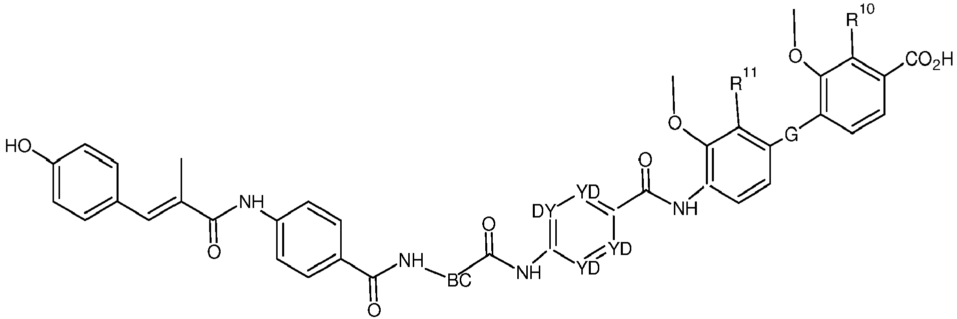 wherein BC, YD, G, R¹¹ and R¹⁰ have the above meaning.

It is to be understood that with Rt and L₁, L₂ as defined for BC there could be two chiral centers here (providing L₁ and L₂ are not the same). Thus diastereoisomers are possible in addition to enantiomers.

In one embodiment of the present compounds according to formulas (1a,1b,1c) the moiety L₁ is a five membered or six membered aromatic heterocycle or 3-7 membered non-aromatic heterocycle, preferably a five membered or six membered aromatic N-heterocycle or non-aromatic N heterocycle that may be substituted or unsubstituted.

In specific embodiments the moiety L₁ is a five membered aromatic N-heterocycle selected from a group comprising substituted or unsubstituted
- pyrroles, imidazoles, pyrazoles, triazoles, tetrazoles;
- pyrazolone, preferably 3H-pyrazol-3-ones, 4H-pyrazol-4-ones, 1,2-dihydro-3H-pyrazol-3-ones, 2,4-dihydro-3H-pyrazol-3-ones, triazolones, preferably 1,2,4-triazol-3-one, imidazolones, pyrrolidones,
- thiadiazoles, preferably 1,3,4-thiadiazoles, thiazoles, isothiazoles, thiazolidinediones; and
- isoxazoles, oxazoles, oxadiazoles (1,3,4-oxadiazoles, 1,2,4-oxadiazoles).

The aromatic five membered heterocyles may be preferably substituted by a C₁-C₆ alkyl moiety, most preferably by a methyl or ethyl moiety. It is most preferred, if the N atom is substituted by a C₁-C₆ alkyl moiety, most preferably by a methyl or ethyl moiety.

In further embodiments of the present compound of formula (1) the moiety L₁ is a five membered non-aromatic N-heterocycle selected from a group comprising substituted or unsubstituted
- pyrrolidines, pyrazolidines,
- hydantoines, imidazolidinones (imidazolidin-4-one), isoxazolidines, oxazolidinones (1,3,-oxazolidin-2-one);
- isothiazolidines, isothiazolinone.

In yet further embodiments the moiety L₁ is a six membered aromatic N-heterocycle selected from a group comprising substituted or unsubstituted pyridines, pyridazines, pyrimidines, pyrazines, triazines and tetrazines.

In still another embodiment of the present compound of formula (1) the moiety L₁ is a six membered non-aromatic N heterocycle selected from a group comprising substituted or unsubstituted piperidines and piperazines or morpholines.

The non-aromatic 5 and 6 membered heterocyles may be preferably substituted by a C₁-C₆ alkyl moiety, most preferably by a methyl or ethyl moiety. It is most preferred, if the N atom is substituted by a C₁-C₆ alkyl moiety, most preferably by a methyl or ethyl moiety. For example, a suitable substituted N-heterocycle may be N-methyl piperidine.

The moiety L₂ may be selected from -H, -OH, -OR^{d}, and -CH₃, -C₂H₆ or -C₃H₇, with R^{d} being substituted or unsubstituted C₁-C₅ alkyl, preferably a C₁-C₃ alkyl.

In a variant Z is being H and Y being CN or -C(=O)NH₂, more preferably Z being H and Y being CN.

In another preferred embodiment of the present compounds n of R¹⁰ₙ and n of R¹¹ₙ is 0, 1, 2, 3 or 4, in particular n of R¹⁰ₙ and n of R¹¹ₙ is 0, 1, 2 or 3, and with each R¹⁰ and with each R¹¹ independently from any other R¹⁰ being selected from -OH, -F, -OCH₃, -OC₂H₅, -OnC₃H₇,-OisoC₃H₇, -OCF₃, -CF₃ or -(CH₂)m-ORₐ,
with Rₐ being selected from hydrogen, -CH₃, -CH₂CH₃, -CH₂CH₂CH₃, -CH₂CH₂CH₂CH₃,-CH(CH₃)₂, -CH₂CH(CH₃)₂, -C(CH₃)₃, -C₆H₅ -CH₂C₆H₅,
with m being selected from 1 or 2,
more particularly with one R¹⁰ or R¹¹ being -OH and the other R¹⁰ or R¹¹ being -OCH₃, - OC₂H₅ or -OiPr respectively.

Particular embodiments of the invention are one the following compounds:
Compound 1: 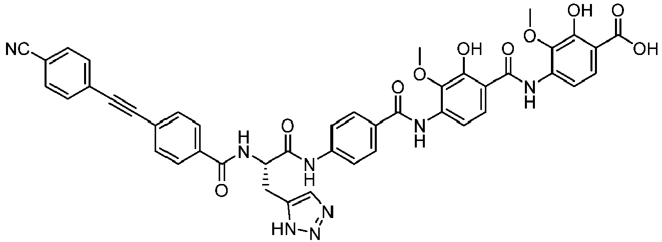
Compound 2a: 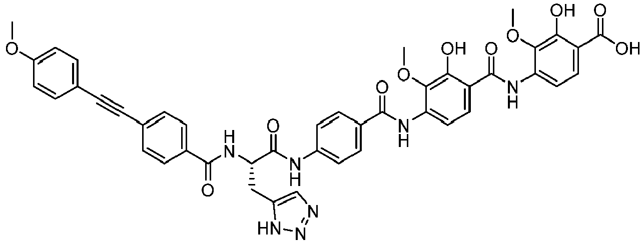
Compound 3: 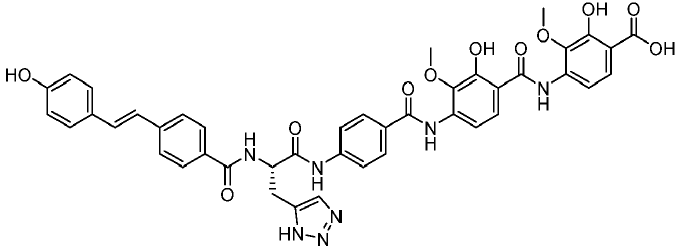
Compound 4: 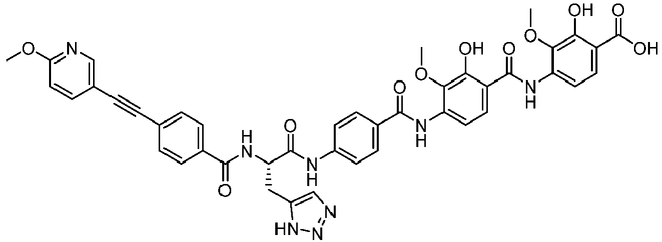
Compound 5: 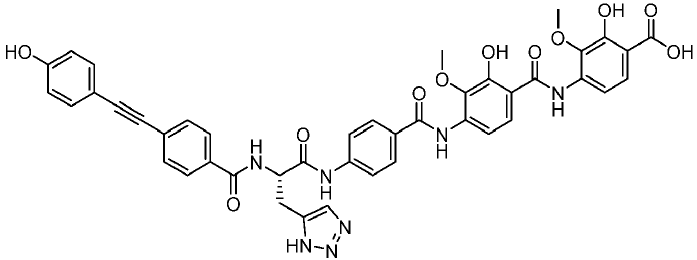
Compound 6: 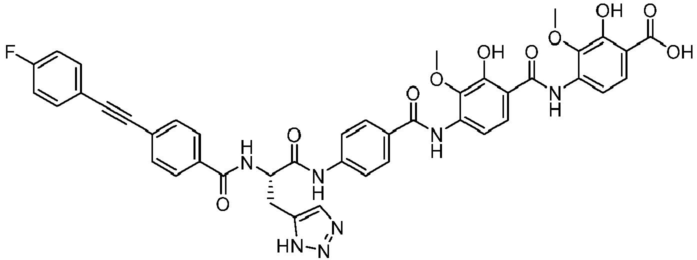
Compound 8: 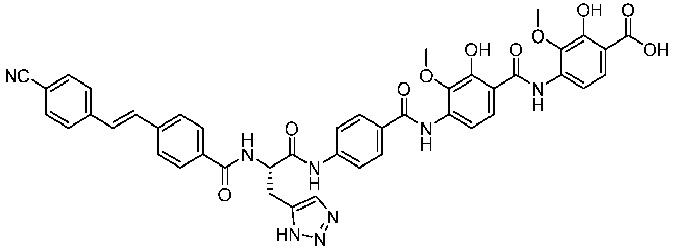
Compound 9: 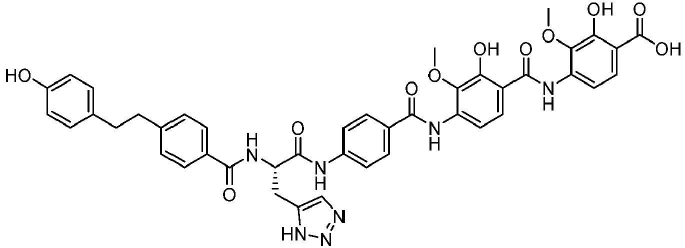
Compound 10: 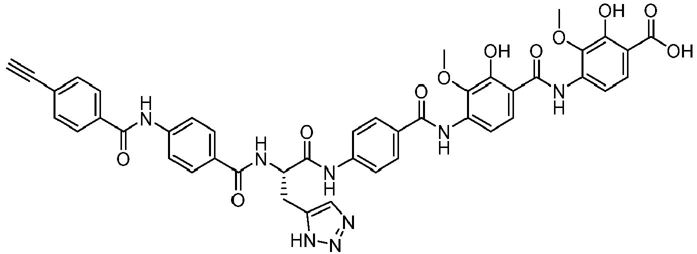
Compound 11: 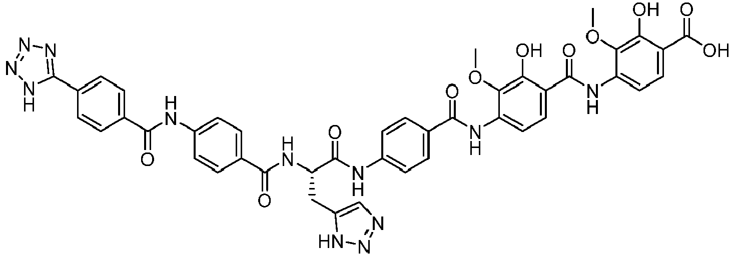
Compound 14 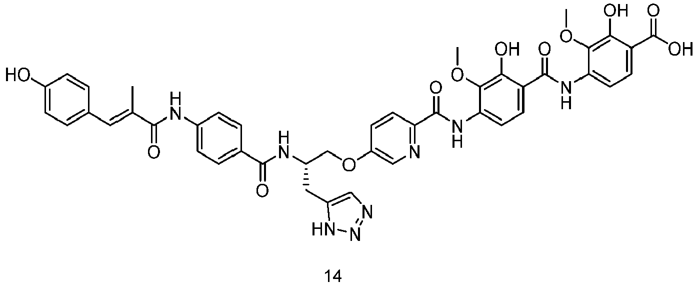
Compound 15: 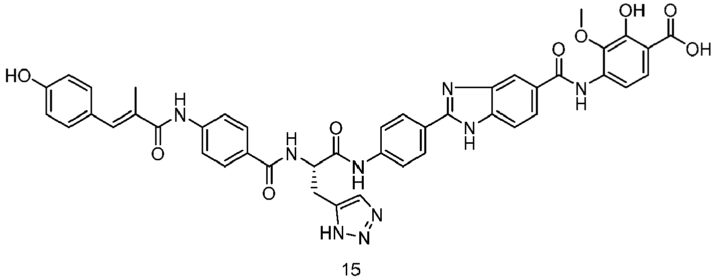
Compound 16: 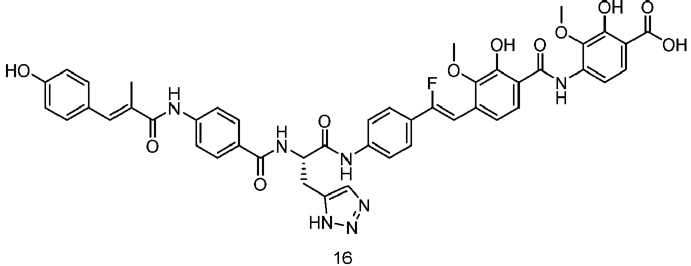
Compound 17: 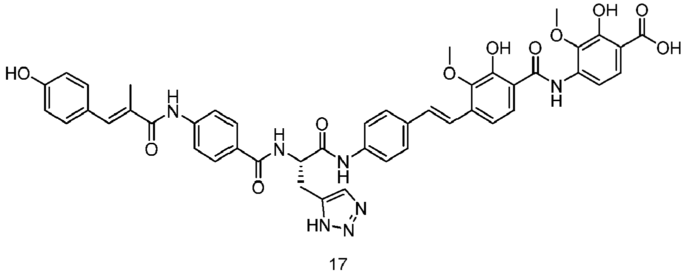
Compound 18: 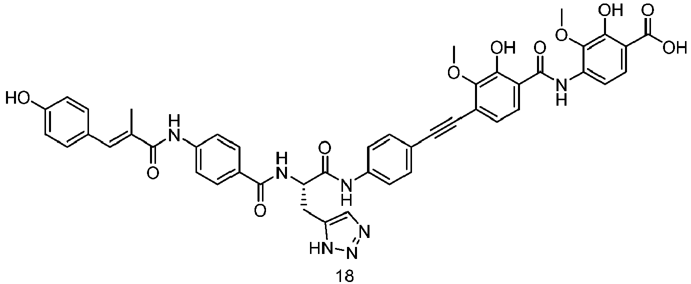
Compound 18b: 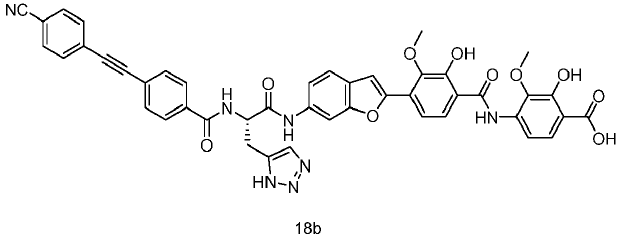
Compound 32: 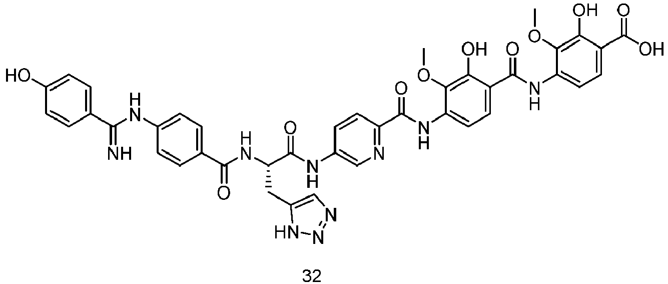
Compound 33:

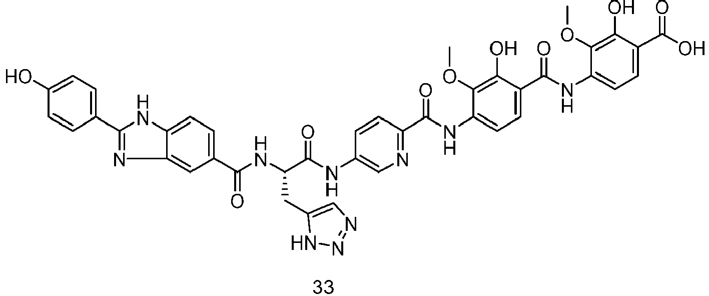

The compounds of the present invention may be used in a method of treatment of diseases, in particular for use in a method of treatment of bacterial infections. For this purpose, the present compounds may be provided in a pharmaceutical acceptable form.

Pharmaceutically acceptable salts of the present compounds mean both their organic and inorganic salts as described in Remington's Pharmaceutical Sciences (17th edition, page 1418 (1985)). Because of the physical and chemical stability and the solubility, preference is given for acidic groups inter alia to sodium, potassium, calcium and ammonium salts; preference is given for basic groups inter alia to salts of maleic acid, fumaric acid, succinic acid, malic acid, tartaric acid, methylsulfonic acid, hydrochloric acid, sulfuric acid, phosphoric acid or of carboxylic acids or sulfonic acids, for example as hydrochlorides, hydrobromides, phosphates, sulfates, methanesulfonates, acetates, lactates, maleates, fumarates, malates, gluconates, and salts of amino acids, of natural bases or carboxylic acids. The preparation of pharmaceutically acceptable salts from compounds of the formula (I) which are capable of salt formation, including their stereoisomeric forms, takes place in a manner known per se. The present compounds form stable alkali metal, alkaline earth metal or optionally substituted ammonium salts with basic reagents such as hydroxides, carbonates, bicarbonates, alcoholates and ammonia or organic bases, for example trimethyl- or triethylamine, ethanolamine, diethanolamine or triethanolamine, trometamol or else basic amino acids, for example lysine, ornithine or arginine. Where the compounds of the formula (I) have basic groups, stable acid addition salts can also be prepared with strong acids. Suitable pharmaceutically acceptable acid addition salts of the compounds of the invention are salts of inorganic acids such as hydrochloric acid, hydrobromic, phosphoric, metaphosphoric, nitric and sulfuric acid, and of organic acids such as, for example, acetic acid, benzenesulfonic, benzoic, citric, ethanesulfonic, fumaric, gluconic, glycolic, isethionic, lactic, lactobionic, maleic, malic, methanesulfonic, succinic, p-toluenesulfonic and tartaric acid. The hydrochloride salt is a preferred salt.

Salts with a pharmaceutically unacceptable anion such as, for example, trifluoroacetate likewise belong within the framework of the invention as useful intermediates for the preparation or purification of pharmaceutically acceptable salts and/or for use in non-therapeutic, for example in vitro, applications.

The present invention furthermore relates to pharmaceutical preparations (or pharmaceutical compositions) which contain an effective amount of at least one of the present compounds and/or its pharmaceutically acceptable salts and a pharmaceutically acceptable carrier, i. e. one or more pharmaceutically acceptable carrier substances (or vehicles) and/or additives (or excipients). The pharmaceuticals can be administered orally, for example in the form of pills, tablets, lacquered tablets, coated tablets, granules, hard and soft gelatine capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The pharmaceutical preparations according to the invention are prepared in a manner known per se and familiar to one skilled in the art, pharmaceutically acceptable inert inorganic and/or organic carrier substances and/or additives being used in addition to the compound(s) of the formula (I) and/or its (their) pharmaceutically acceptable salts and/or its (their) prodrugs. For the production of pills, tablets, coated tablets and hard gelatine capsules it is possible to use, for example, lactose, corn starch or derivatives thereof, talc, stearic acid or its salts, etc. Carrier substances for soft gelatine capsules and suppositories are, for example, fats, waxes, semisolid and liquid polyols, natural or hardened oils, etc. Suitable carrier substances for the production of solutions, for example injection solutions, or of emulsions or syrups are, for example, water, saline, alcohols, glycerol, polyols, sucrose, invert sugar, glucose, vegetable oils, etc. Suitable carrier substances for microcapsules, implants or rods are, for example, copolymers of glycolic acid and lactic acid. The pharmaceutical preparations normally contain about 0.5 to about 90 % by weight of the present compounds and/or their pharmaceutically acceptable salts and/or their prodrugs. The amount of the active ingredient of the formula (I) and/or its pharmaceutically acceptable salts and/or its prodrugs in the pharmaceutical preparations normally is from about 0.5 to about 1000 mg, preferably from about 1 to about 500 mg.

A prodrug within the meaning of the present invention is a precursor chemical compound of a biological active compound of the present invention. Instead of administering the active compound or drug, a prodrug might be used instead to improve the absorption, distribution, metabolization and excretion. Prodrugs are often designed to improve bioavailability when a drug itself is poorly absorbed from the gastrointestinal tract. A prodrug may also be used to improve the selectively of the drug. This reduces adverse or unintended effects of a drug, especially important in treatments like chemotherapy, which can have severe unintended and undesirable side effects.

In addition to the active compound according to the invention and/or their pharmaceutically acceptable salts and to carrier substances, the pharmaceutical preparations can contain one or more additives such as, for example, fillers, disintegrants, binders, lubricants, wetting agents, stabilizers, emulsifiers, preservatives, sweeteners, colorants, flavourings, aromatizers, thickeners, diluents, buffer substances, solvents, solubilizers, agents for achieving a depot effect, salts for altering the osmotic pressure, coating agents or antioxidants. They can also contain two or more of the present compounds and/or their pharmaceutically acceptable salts. In case a pharmaceutical preparation contains two or more of the present compounds the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in the present compounds allows a great deal of control over the biological and physico-chemical properties of the compounds and thus allows the selection of such desired compounds. Furthermore, in addition to at least one compound and/or its pharmaceutically acceptable salts, the pharmaceutical preparations can also contain one or more other therapeutically or prophylactically active ingredients. When using the present compounds the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily dose for achieving the desired results in an adult weighing about 75 kg is from about 0.01 to about 100 mg/kg, preferably from about 0.1 to about 50 mg/kg, in particular from about 0.1 to about 10 mg/kg, (in each case in mg per kg of body weight). The daily dose can be divided, in particular in the case of the administration of relatively large amounts, into several, for example 2, 3 or 4, part administrations. As usual, depending on individual behaviour it may be necessary to deviate upwards or downwards from the daily dose indicated.

The compounds of the invention may also exist in various polymorphous forms, for example as amorphous and crystalline polymorphous forms. All polymorphous forms of the compounds of the invention belong within the framework of the invention and are a further aspect of the invention.

The compounds of the present invention may be present as optical isomers or as mixtures thereof. The invention relates both to the pure isomers and all possible isomeric mixtures and is hereinafter understood as doing so, even if stereochemical details are not specifically mentioned in every case. Enantiomeric mixtures of compounds of the general formula 1, which are obtainable by the process or any other way, may be separated in known manner - on the basis of the physical-chemical differences of their components - into pure enantiomers, for example by fractional crystallisation, distillation and/or chromatography, in particular by preparative HPLC using a chiral HPLC column.

According to the invention, apart from separation of corresponding isomer mixtures, generally known methods of diastereoselective or enantioselective synthesis can also be applied to obtain pure diastereoisomers or enantiomers, e.g. by carrying out the method described hereinafter and using educts with correspondingly suitable stereochemistry.

It is advantageous to isolate or synthesize the biologically more active isomer, provided that the individual compounds have different biological activities.

The present invention is explained in more detail by means of the following examples.

### Methods of synthesis

One general procedure for the synthesis of albicidin-derivatives with variations to amide bonds may comprise the steps according to the following procedure:
Compound 1 is synthesized in a multistep synthesis route as follows:

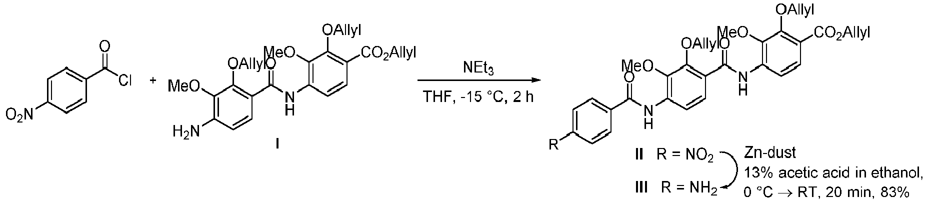

### Preparation of compound II:

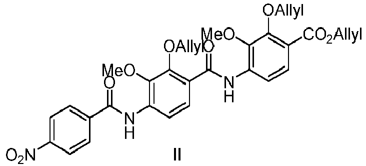

The literature known amine I (1 eq, 11.87 mmol, 5.56 g) was dissolved in anhydrous THF (24 mL) and triethylamine (3.01 eq, 35.71 mmol, 4.95 mL) was added. The solution was cooled to -15 °C and 4-Nitrobenzoylchloride (1.51 eq, 17.88 mmol, 3.32 g) was added in one portion. The reaction mixture was stirred for 20 minutes and diluted with diethyl ether (22 ml). The solid was filtered, washed with diethyl ether (3 x 50 ml) and dried *in vacuo* to yield II (7.30 g, 0.012 mmol, -quant.) as a yellow solid.
**¹H NMR (DMSO-d₆, 400 MHz): δ** (ppm) = 10.65 (s, 1 H), 10.27 (s, 1 H), 8.35 - 8.41 (m, 2 H), 8.32 (d, *J* = 8.8 Hz, 1 H), 8.17 - 8.22 (m, 2 H), 7.83 (q, *J* = 8.8 Hz, 2 H), 7.57 (d, *J* = 8.8 Hz, 1 H), 5.98 - 6.17 (m, 3 H), 5.35 - 5.44 (m, 3 H), 5.22 - 5.32 (m, 3 H), 4.75 - 4.82 (m, 4 H), 4.52 - 4.56 (m, 2 H), 3.93 (s, 3 H), 3.90 (s, 3 H).
**¹³C NMR (DMSO-d₆, 101 MHz): δ** (ppm) = 164.5, 164.4, 162.4, 151.1, 149.7, 149.3, 145.1, 142.5, 139.9, 136.5, 135.9, 134.0, 132.7, 132.6, 129.5, 126.3, 125.4, 123.8, 123.6, 120.3, 120.1, 119.6, 118.1, 117.9, 114.9, 75.1, 74.6, 65.1, 61.0, 60.9.
**HRMS (ESI):** *m*/*z* calc. for C₃₂H₃₁N₃O₁₀ [M+H]⁺: 618.2082; found 618.2079

### Preparation of compound III:

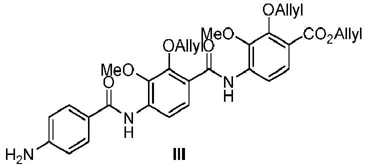

Compound II (1 eq, 12.84 mmol, 7.30 g) was suspended in a mixture of ethanol (800 ml) and acetic acid (100 ml) and cooled to 0 °C. Zinc dust (33.80 g) was added portion wise. After 20 min the reaction was proven to be complete (verified by TLC-control). The solid was filtered and washed with DCM (3 x 100 ml). The combined liquids were evaporated to dryness. The residue was taken up in DCM (300 ml) and saturated aqueous NaHCO₃-Solution (300 ml). The aqueous phase was further extracted twice with DCM (2 x 100 ml). The combined organic fractions were washed successively with saturated aqueous NaHCO₃-Solution (1 x 300 ml), distilled water (1 x 300 ml) and brine (1 x 300 ml), dried over Na₂SO₄ and evaporated to obtain III (5.79 g, 9.85 mmol, 83%) as a yellow solid.
**¹H NMR (DMSO-d₆, 400 MHz): δ** (ppm) = 10.65 (s, 1 H), 9.19 (s, 1 H), 8.34 (d, *J* = 8.8 Hz, 1 H), 8.01 (d, *J* = 8.8 Hz, 1 H), 7.79 (d, *J* = 8.8 Hz, 1 H), 7.68 - 7.74 (m, 2 H), 7.57 (d, *J* = 9.0 Hz, 1 H), 6.59 - 6.65 (m, 2 H), 5.98 - 6.18 (m, 3 H), 5.89 (s, 2 H), 5.40 (tdd, J = 11.5, 5.6, 1.5 Hz, 3 H), 5.21 - 5.32 (m, 3 H), 4.75 - 4.83 (m, 4 H), 4.54 (d, *J* = 5.8 Hz, 2 H), 3.93 (s, 3 H), 3.92 (s, 3 H).
**¹³C NMR (DMSO-d₆, 101 MHz): δ** (ppm) = 165.0, 164.4, 162.4, 152.7, 151.1, 149.4, 143.3, 142.4, 137.2, 136.6, 134.0, 132.7, 132.6, 129.4, 126.3, 125.6, 121.7, 120.2, 120.1, 120.0, 118.1, 117.8, 117.5, 114.8, 112.7, 75.1, 74.5, 65.1, 61.0, 60.9.
**HRMS (ESI):** *m*/*z* calc. for C₃₂H₃₃N₃O₈ [M+H]⁺: 588.2340 ; found 588.2343

### Preparation of compound IV:

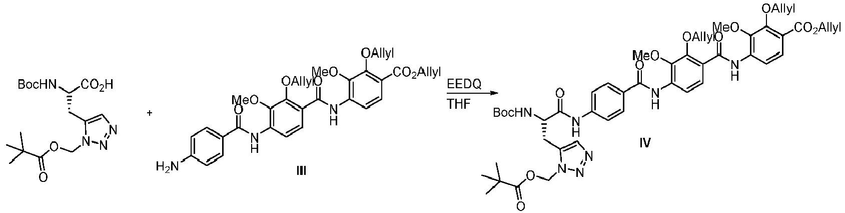

Literature known Boc-**β**-(1-pivaloyloxymethyl)-1,2,3-triazol-4-yl)-Alanine (1.46 eq, 3.99 mmol, 1.48 g) was dissolved in THF (20 ml) and cooled to 0 °C. *N*-Ethoxycarbonyl-2-ethoxy-1,2-dihydroqinoline (EEDQ) (3.00 eq, 8.20 mmol, 2.03 g) was added and after 5 minutes compound III (1 eq, 2.72 mmol, 1.6 g) was added. The reaction mixture was slowly warmed to room temperature and stirred for 16 h. All volatiles were removed *in vacuo* and the residue was taken up in ethyl acetate (100 ml). The organic fraction was washed with saturated aqueous NaHCO₃-Solution (3 x 50 ml) and brine (1 x 50 ml), dried over Na₂SO₄ and evaporated. The residue was purified *via* flash chromatography on silica gel eluting with 1-15% acetone in DCM. Compound **IV** (1.90 g, 2.02 mmol, 74 %) was obtained as a light-yellow solid.
**¹H NMR** (DMSO-d₆, 500MHz): **δ** = 10.65 (s, 1 H), 10.41 (s, 1 H), 9.63 (s, 1 H), 8.33 (d, *J*=8.7 Hz, 1 H), 7.92 - 7.99 (m, 4 H), 7.74 - 7.84 (m, 3 H), 7.57 (d, J=8.7 Hz, 1 H), 7.20 (m, 1 H), 6.29 (s, 2 H), 5.99 - 6.16 (m, 3 H), 5.22 - 5.45 (m, 6 H), 4.81 (d, J=6.1 Hz, 2 H), 4.77 (d, J=5.5 Hz, 2 H), 4.54 (d, J=5.6 Hz, 3 H), 3.93 (d, *J*=6.1 Hz, 6 H), 2.96 - 3.16 (m, 2 H), 1.26 - 1.38 (m, 9 H), 1.09 ppm (s, 9 H)
**¹³C NMR** (DMSO-d₆, 126MHz): **δ** = 176.4, 170.7, 164.8, 164.4, 162.4, 155.3, 151.1, 149.5, 144.2, 143.4, 142.5, 142.2, 136.5, 133.9, 132.7, 132.6, 128.7, 128.5, 126.3, 125.5, 124.1, 122.7, 120.3, 120.1, 118.7, 118.6, 118.1, 117.8, 114.8, 78.3, 75.1, 74.5, 69.8, 65.1,61.0, 60.9, 54.9, 38.1, 28.1, 26.4 ppm
**HRMS** (ESI): m/z calc. for C₄₈H₅₇N₇O₁₃ [M+H]⁺ 940.4087, found 940.4088.

### Preparation of compound V:

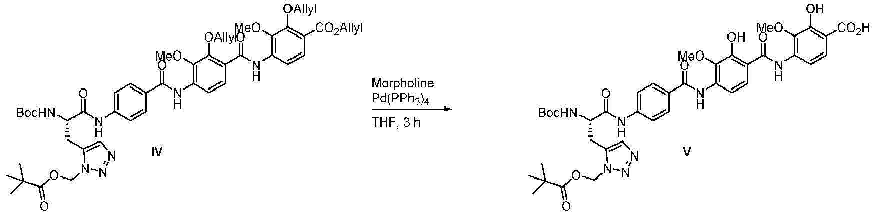

Tetrapeptide **IV** (1 eq, 2.00 mmol, 1.88 g) was dissolved in THF (5 ml) and morpholine (20 eq, 40.00 mmol, 3.48 g) and *tetrakis*(triphenylphosphin)palladium(0) (0.3 eq, 0.60 mmol, 693 mg) were added. The mixture was stirred for 2.5 h shielded from light. All volatiles were removed *in vacuo* and the residue was purified via flash chromatography on C-18-material eluting with 5 to 50% acetonitrile in water. Compound V (1.24 g, 1.51 mmol, 76 %) was obtained as a white solid.
**¹H NMR** (DMSO-d₆, 500MHz): **δ** = 11.51 (s, 1 H), 11.16 (s, 1 H), 9.64 (s, 1 H), 8.05 (d, *J*=9.0 Hz, 1 H), 7.96 (d, *J*=8.9 Hz, 3 H), 7.81 (d, *J*=8.9 Hz, 1 H), 7.76 (d, *J*=8.7 Hz, 2 H), 7.59 (dd, J=8.9, 3.8 Hz, 2 H), 7.17 - 7.20 (m, 1 H), 6.29 (s, 2 H), 4.38 - 4.44 (m, 1 H), 3.92 (s, 3 H), 3.78 (s, 3 H), 2.97 - 3.15 (m, 2 H), 1.26 - 1.38 (m, 9 H), 1.09 ppm (s, 9 H)
**¹³C NMR** (DMSO-d₆, 126MHz): **δ** = 176.4, 172.0, 164.8, 164.4, 163.3, 154.3, 149.7, 146.2, 143.4, 142.2, 140.1, 137.8, 136.1, 135.9, 128.7, 128.6, 128.3, 125.4, 124.1, 118.7, 116.1, 114.8, 110.3, 109.0, 78.3, 69.8, 60.5, 60.2, 59.7, 38.1, 28.1, 26.4 ppm
**HRMS** (ESI): m/z calc. for C₃₉H₄₅N₇O₁₃ [M-H]⁻ 818.3003, found 818.3009.

### Preparation of compound VI:

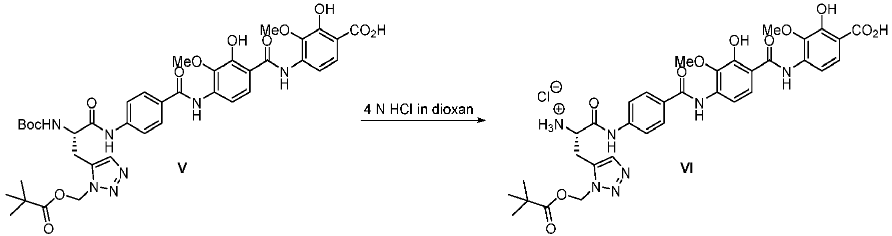

Tetrapeptide **V** (1.00 eq, 1.51 mmol, 1.24 g) was dissolved 4 N HCI in dioxane and stirred for 1 hour. The solvent was evaporated in vacuo and the product **VI** (1.13 g, 1.50 mmol, quant.) was obtained as white solid. Compound **VI** was used in the next step without further characterization.
**HRMS** (ESI): m/z calc. for C₃₄H₃₇N₇O₁₁ [M+H]⁺: 720.2624, found: 720.2624.

### Preparation of active ester XI

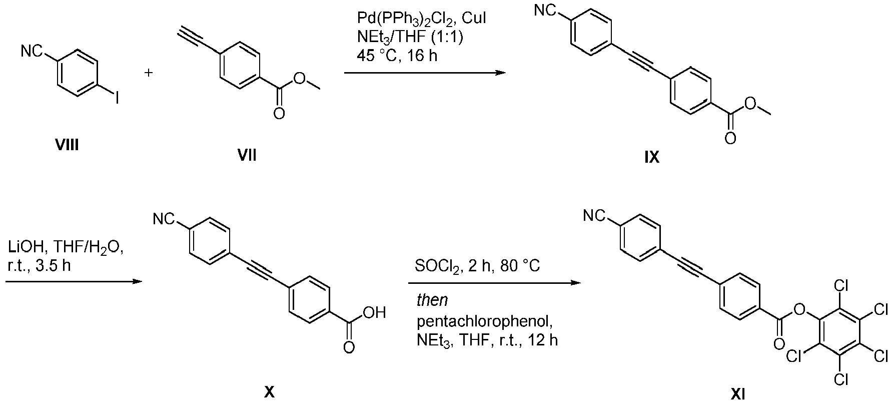

### Preparation of compound IX

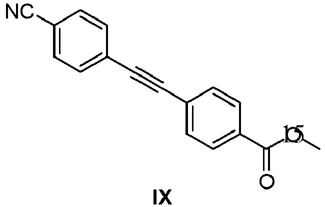

Commercially available compound **VIII** (1.00 eq, 11.4 mmol, 2.60 g) and NEt₃ (10 mL) were dissolved in THF (10 mL). Compound **VII** (1.20 eq, 13.6 mmol, 2.18 g) was added to the reaction mixture at 25 °C. After 16 h at 45 °C the solvent was removed *in vacuo.* The resulting solid was diluted with CH₂Cl₂ (100 mL) and quenched with 1 N HCl. The water phase was extracted with CH2CI2 (3 x) the combined organic layers were washed with 1 N HCI (2 x), Brine (1 x) and concentrated *in vacuo.* The crude product was purified by column chromatography (cyclohexane: ethylacetate; 20:1). Compound **IX** was obtained as a colorless solid (2.29 g, 8.76 mmol, 77 %).
**¹H NMR** (DMSO-d₆ ,500MHz): δ 8.01 (d, *J* = 8.4 Hz, 2H), 7.92 (d, *J* = 8.4 Hz, 2H), 7.78 (d, *J* = 8.4 Hz, 2H), 7.74 (d, *J* = 8.3 Hz, 2H), 3.88 (s, 3H).
**¹³C NMR** (DMSO-d₆ ,126MHz): ^{z}δ 165.53, 132.65, 132.32, 131.95, 129.93, 129.46, 126.53, 126.12, 118.33, 111.45, 92.08, 90.57, 52.36.
**HRMS** (ESI): m/z calc. for C₁₇H₁₁NO₂ [M+H]⁺: 262.0863, found: 262.0861.

### Preparation of compound X

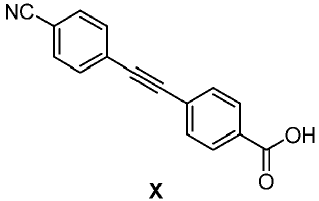

Methylester **IX** (1.00 eq, 3.83 mmol, 1.00 g) was dissolved in THF (20 mL). A solution of LiOH (2.00 eq., 7.65 mmol, 321 mg) in water (20 mL) was added. After 3.5 h at r.t. the reaction mixture was concentrated *in vacuo* and diluted with H₂O (10 mL). The product was precipitated with 6 N HCl, filtered and washed with H₂O. Compound **X** (908 mg, 3.67 mmol, 95 %) was obtained as a light gray solid.
**¹H NMR** (500 MHz, DMSO-*d*₆) δ 7.99 (d, *J* = 8.4 Hz, 2H), 7.90 (d, *J* = 8.4 Hz, 2H), 7.76 (d, *J* = 8.4 Hz, 2H), 7.70 (d, *J* = 8.4 Hz, 2H).
**¹³C NMR** (126 MHz, DMSO) δ 166.61, 132.63, 132.30, 131.79, 131.32, 129.59, 126.65, 125.64, 118.36, 111.39, 92.32, 90.25.
**HRMS** (ESI): m/z calc. for C₁₆H₉NO₂ [M-H]⁻: 246.0561, found: 246.0556.

### Preparation of compound XI

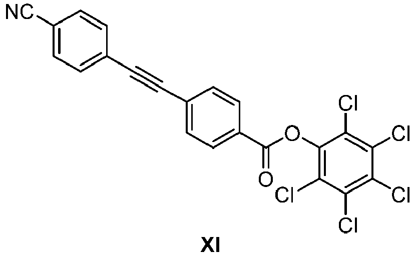

Compound **X** (1 eq, 2.02 mmol, 500 mg) was dissolved in thionyl chloride (10eq., 20.2 mmol, 1.45 mL) and refluxed for 2 h at 80 °C. All volatiles were removed *in vacuo* and the obtained acid chloride was dissolved in THF (dry, 2 mL). The solution was cooled to 0 °C, then pentachlorophenol (1.10 eq, 2.22 mmol, 592 mg) and triethylamine (2.00 eq., 4.04 mmol, 0.564 mL) was added in one portion. The reaction mixture was stirred for 12 h. The solvent was removed *in vacuo* and the crude product purified by column chromatography (cyclohexane: ethyl acetate; 15:1). Active ester **XI** was obtained as a colorless solid (520 mg, 1.05 mmol, 52 %).
**¹H NMR** (500 MHz, DMSO-*d*₆) δ 8.28 (d, J= 8.3 Hz, 2H), 7.96 (d, J= 8.3 Hz, 2H), 7.91 (d, *J* = 8.4 Hz, 2H), 7.84 (d, *J* = 8.3 Hz, 2H).
Due to the low solubility of the compound, no ¹³C-Data were recorded.
**HRMS** (ESI): m/z calc. C₂₂H₈Cl₅NO₂ [M+H]⁺: 495.9041, Mass not found.

### Preparation of compound 1:

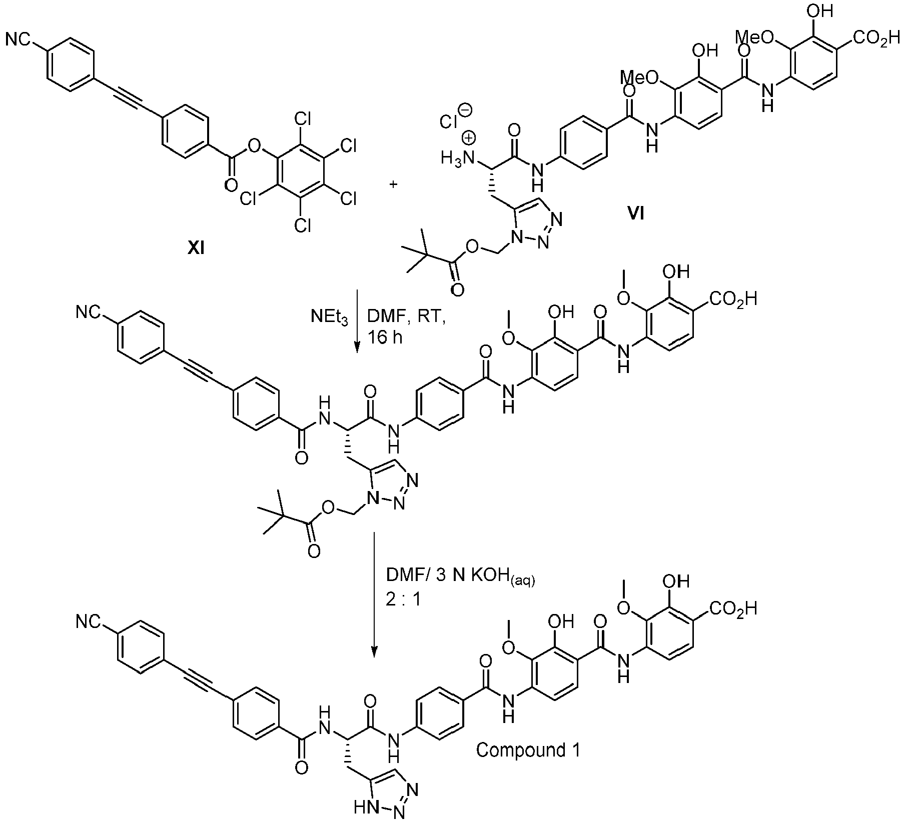

The tetrapeptide **VI** (60 mg, 93 µmol, 1.0 equiv) was dissolved in DMF (2 ml) and triethylamine (8 eq, 0.75 mmol, 0.1 mL) was added. After adding the active ester (1.10 eq, 0.102 mmol, 53.0 mg), the mixture was stirred for 16 h. Then cooled to 0 °C and 3 N KOH_{(aq)} (1 ml) was added dropwise. After 15 min of stirring, 550 µl of 6 N HCl_{(aq)} were added dropwise. The resulting mixture was evaporated to dryness. The residue was purified *via* prep HPLC. Compound 1 (5 mg, 0.006 mmol, 5%) was obtained as a white fluffy solid.
**¹H NMR** (400 MHz, DMSO-*d*₆) **δ** 11.54 (s, 1H), 11.17 (s, 1H), 10.56 (s, 1H), 9.68 (s, 1H), 9.01 (d, *J* = 8.03 Hz, 1H), 7.77-7.83 (m, 5H), 7.73 (d, *J* = 8.53 Hz, 2H), 7.60 (d, *J* = 3.51 Hz, 1H), 7.57 (d, *J* = 3.76 Hz, 1H), 4.88-4.99 (m, 1H), 3.92 (s, 3H), 3.78 (s, 3H). **¹³C NMR** (101 MHz, DMSO-*d*₆) **δ** 132.9 (Ar), 132.7 (Ar), 132.0 (Ar), 128.8 (Ar), 126.3 (Ar), 119.1 (Ar), 115.3 (Ar), 110.3 (Ar), 60.8 (OMe), 60.5 (OMe). **HRMS (ESI):** *m*/*z* calculated for C₄₄H₃₄N₈O₁₀ [M+H]⁺ 835.2471, found 835.2469 (deviation -0.2 ppm), *t*_{R} = 9.38 min.

The following compounds are obtained in an analog synthesis procedures.

### Compound 2a:

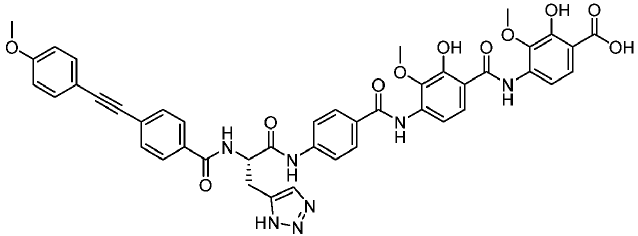

**¹H NMR** (400 MHz, DMSO-*d*₆) **δ** 11.54 (s, 1H), 11.19 (s, 1H), 10.55 (s, 1H), 9.69 (s, 1H), 8.95 (d, *J* = 7.78 Hz, 1H), 8.06 (d, *J* = 9.04 Hz, 1H), 7.97 (d, *J* = 8.78 Hz, 2H), 7.92 (d, *J* = 8.53 Hz, 2H), 7.75-7.84 (m, 3H), 7.63 (d, *J* = 8.53 Hz, 2H), 7.56-7.61 (m, 2H), 7.51-7.56 (m, 2H), 6.98-7.04 (m, 2H), 4.88-4.97 (m, 1H), 3.91 (s, 3H), 3.80 (s, 3H), 3.78 (s, 3H), 3.19-3.35 (m, *J =* 5.52 Hz, 2H). **¹³C NMR** (101 MHz, DMSO-*d*₆) **δ** 133.4 (Ar), 131.4 (Ar), 129.0 (Ar), 128.1 (Ar), 128.1 (Ar), 125.8 (Ar), 125.6 (Ar), 119.1 (Ar), 115.2 (Ar), 114.9 (Ar), 110.8 (Ar), 60.8 (OMe), 60.8 (OMe), 55.7 (OMe), 54.5 (α-C), 27.6 (β-C). **HRMS (ESI):** *m*/*z* calculated for C₄₄H₃₇N₇O₁₁ [M+H]⁺ 840.2624, found 840.2626 (deviation +0.2 ppm), *t*_{R} = 9.38 min.

### Compound 3:

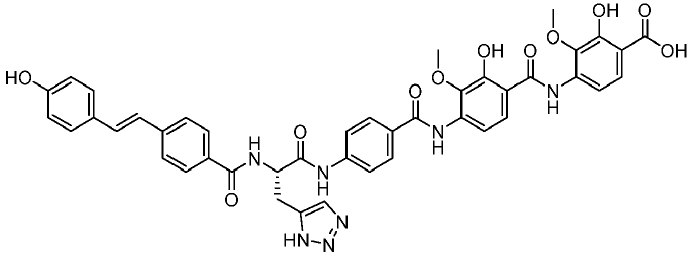

**¹H NMR** (500 MHz, DMSO-*d*₆) δ 10.82 (br s, 1H), 10.59 (s, 1H), 9.61 (s, 1H), 8.79- 8.84 (m, 1H), 7.97 (d, J=9.0 Hz, 2H), 7.88 (d, *J*=8.4 Hz, 2H), 7.80 (br d, *J*=8.9 Hz, 3H), 7.66-7.70 (m, 1H), 7.60-7.66 (m, 2H), 7.55 (d, *J*=8.9 Hz, 1H), 7.41-7.51 (m, 3H), 7.28 (d, *J*=16.5 Hz, 1H), 7.07 (d, *J*=16.5 Hz, 1H), 6.77-6.84 (m, 2H), 4.90-4.96 (m, 1H), 3.86 (s, 3 H), 3.79 (s, 3 H). **¹³C NMR** (126 MHz, DMSO-*d*₆) **δ** 130.7 (Ar-C*H*), 128.9, 128.4, 128.2, 125.9, 125.2, 125.0, 124.7 (CH-Ar), 124.4, 119.0 (Ar), 115.9 (Ar), 114.6 (Ar), 107.8 (Ar), 60.6 (OMe), 59.8 (OMe), 54.6 (α-C), 27.5 (β-C). **HRMS (ESI):** *m*/*z* calculated for C₄₃H₃₇N₇O₁₁ [M+H]⁺ 828.2624, found 828.2628 (deviation -0.4 ppm), *t*_{R} = 8.11 min.

### Compound 4:

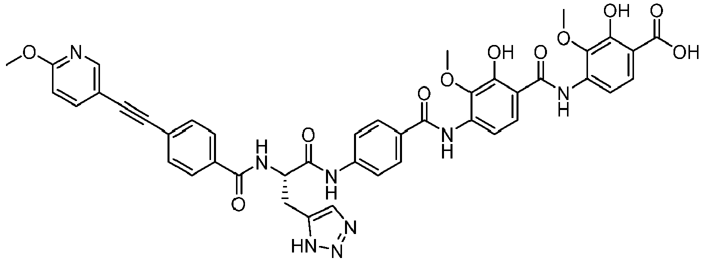

**¹H NMR** (400 MHz, DMSO-*d*₆) δ 11.56 (br. s., 1H), 11.20 (s, 1H), 10.56 (s, 1H), 9.69 (s, 1H), 8.98 (d, *J* = 7.53 Hz, 1H), 8.44 (d, *J* = 2.01 Hz, 1H), 8.06 (d, *J* = 8.78 Hz, 1H), 7.87-8.01 (m, 5H), 7.76-7.84 (m, 3H), 7.63-7.72 (m, 3H), 7.60 (d, *J* = 5.52 Hz, 1H), 7.58 (d, *J* = 5.52 Hz, 1H), 6.91 (d, J= 8.78 Hz, 1H), 4.88-4.99 (m, 1H), 3.91 (s, 3H), 3.90 (s, 3H), 3.78 (s, 3H), 3.20-3.37 (m, 2H). **¹³C NMR** (101 MHz, DMSO-*d*₆) δ 150.4 (Ar), 142.0 (Ar), 131.5 (Ar), 128.7 (Ar), 126.0 (Ar), 125.9 (Ar), 119.2 (Ar), 115.2 (Ar), 111.2 (Ar), 110.9 (Ar), 60.8 (OMe), 54.7 (OMe), 54.3 (α-C), 54.0 (OMe), 27.1 (β-C). **HRMS (ESI):** *m*/*z* calculated for C₄₃H₃₆N₈O₁₁ [M+H]⁺ 841.2576, found 841.2584 (deviation +1.0 ppm), *t*_{R} = 8.63 min.

### Compound 5:

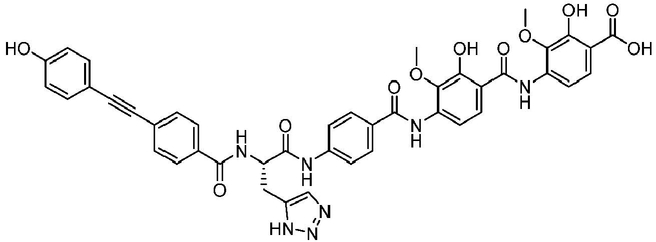

**¹H NMR** (500 MHz, DMSO-*d*₆) δ 11.52 (s, 1H), 11.17 (s, 1H), 10.52 (s, 1H), 9.99 (s, 1H), 9.66 (s, 1H), 8.92 (d, *J* = 7.32 Hz, 1H), 8.06 (d, *J* = 8.85 Hz, 1H), 7.98 (d, *J* = 8.70 Hz, 2H), 7.91 (d, *J* = 8.39 Hz, 2H), 7.75-7.84 (m, 3H), 7.56-7.64 (m, 4H), 7.41 (d, *J* = 8.54 Hz, 2H), 6.82 (d, *J* = 8.70 Hz, 2H), 4.89-4.99 (m, *J* = 6.26 Hz, 1H), 3.92 (s, 3H), 3.79 (s, 3H), 3.21-3.41 (m, 2H). **¹³C NMR** (126 MHz, DMSO-*d*₆) δ 133.7 (Ar), 131.4 (Ar), 129.1 (Ar), 128.3 (Ar), 126.1 (Ar), 119.2 (Ar), 116.3 (Ar), 115.3 (Ar), 110.7 (Ar), 60.6 (OMe), 61.0 (OMe), 54.8 (α-C), 29.4 (β-C). **HRMS (ESI):** *m*/*z* calculated for C₄₃H₃₅N₇O₁₁ [M+H]⁺ 826.2467, found 826.2454 (deviation -1.6 ppm), *t*_{R} = 8.38 min.

### Compound 6:

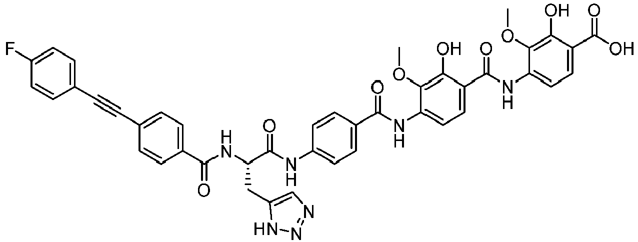

**¹H NMR** (400 MHz, DMSO-*d*₆) δ 11.55 (s, 1H), 11.19 (s, 1H), 10.56 (s, 1H), 9.69 (s, 1H), 8.98 (d, *J* = 7.53 Hz, 1H), 8.06 (d, *J* = 9.03 Hz, 1H), 7.96 (dd, *J* = 8.53, 15.56 Hz, 4H), 7.76-7.85 (m, 3H), 7.63-7.73 (m, 5H), 7.59 (dd, *J* = 5.40, 8.91 Hz, 2H), 7.30 (t, *J* = 8.91 Hz, 2H), 4.88-4.99 (m, 1H), 3.92 (s, 3H), 3.78 (s, 3H), 3.19-3.37 (m, 2H). **¹³C NMR** (101 MHz, DMSO-*d*₆) δ 134.2 (Ar), 132.7 (Ar), 129.4 (Ar), 128.1 (Ar), 126.1 (Ar), 125.8 (Ar), 119.1 (Ar), 116.5 (Ar), 115.3 (Ar), 110.6 (Ar), 60.8 (OMe), 60.6 (OMe), 54.7 (α-C), 27.6 (β-C). **HRMS (ESI):** *m*/*z* calculated for C₄₃H₃₄FN₇O₁₀ [M+H]⁺ 828.2424, found 828.2427 (deviation +0.4 ppm) *t*_{R} = 9.04 min.

### Compound 8:

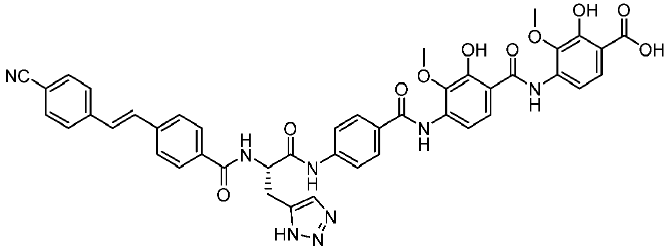

**¹H NMR** (500 MHz, DMSO-*d*₆) δ 11.54 (br s, 1H), 11.17 (s, 1H), 10.58 (s, 1H), 9.67 (s, 1H), 8.86-8.91 (m, 1H), 8.02-8.08 (m, 1H), 7.97 (d, *J*=8.7 Hz, 2H), 7.93 (d, *J*=8.1 Hz, 2H), 7.77-7.87 (m, 8H), 7.75 (d, *J*=8.4 Hz, 2H), 7.69 (br s, 1 H), 7.60-7.55 (m, 2H), 7.51 (d, *J*=*1*3.9 Hz, 2H), 4.90-4.97 (m, 1H), 3.91 (s, 3H), 3.77-3.79 (m, 3H), 3.77-3.79 (m, 3 H). **¹³C NMR** (126 MHz, DMSO-*d*₆) δ 133.1, 131.2 (Ar-C*H*), 129.8 (CH-Ar), 129.6 (Ar), 128.6 (Ar), 127.4 (Ar), 127.4 (Ar), 126.1 (Ar), 119.3 (Ar), 115.3 (Ar), 110.8 (Ar), 61.1 (OMe), 61.0 (OMe), 54.8 (α-C), 27.9 (β-C). **HRMS (ESI):** *m*/*z* calculated for C₄₄H₃₆N₈O₁₀ [M+H]⁺ 837.2627, found 837.2631 (deviation -0.4 ppm), *t*_{R} = 8.87 min.

### Compound 9:

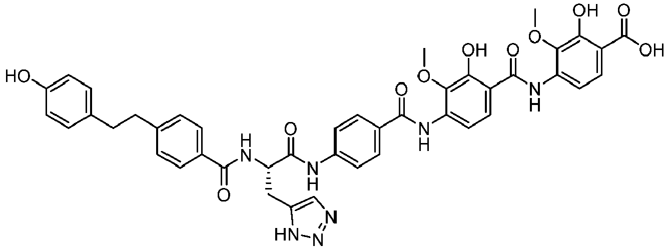

**¹H NMR** (400 MHz, DMSO-*d*₆) δ 11.52 (s, 1H), 11.16 (s, 1H), 10.49 (s, 1H), 9.65 (s, 1H), 9.01-9.22 (m, 1H), 8.68-8.75 (m, 1H), 8.05 (d, *J*=8.8 Hz, 1H), 7.93-8.00 (m, 2H), 7.73-7.82 (m, 5H), 7.63-7.70 (m, 1H), 7.54-7.63 (m, 2H), 7.29 (d, *J*=8.0 Hz, 2H), 6.99 (d, *J*=8.5 Hz, 2H), 6.65 (d, *J*=8.3 Hz, 2H), 4.85-4.95 (m, 1H), 3.92 (s, 3H), 3.78 (s, 3H), 3.06-3.14 (m, 2H), 2.84-2.91 (m, 2H), 2.74-2.81 (m, 2H). **¹³C NMR** (101 MHz, DMSO) δ 129.8 (Ar), 129.4 (Ar), 119.2 (Ar), 127.9 (Ar), 128.8 (Ar), 115.6 (Ar), 60.5 (Ar), 60.9 (Ar), 46.1 (CH2), 37.4 (CH2), 36.1 (CH2). **HRMS (ESI):** *m*/*z* calculated for C₄₃H₃₉N₇O₁₁ [M+H]⁺ 830.2780, found 830.2780 (deviation 0 ppm), *t*_{R} = 8.23 min.

### Compound 10:

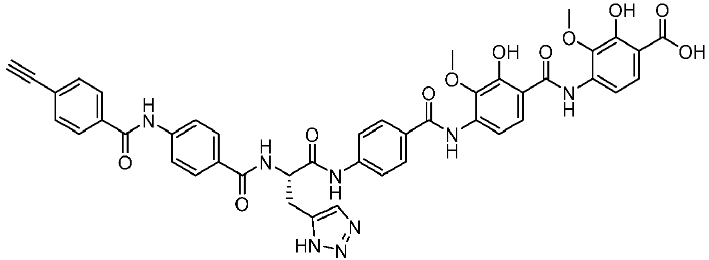

**¹H NMR** (400 MHz, DMSO-*d*₆): δ 11.54 (s, 1H), 11.19 (s, 1H), 10.55 (s, 1H), 10.54 (s, 1H), 9.69 (s, 1H), 8.76 (d, *J* = 7.53 Hz, 1H), 8.06 (d, *J* = 9.03 Hz, 1H), 7.98 (dd, *J* = 6.65, 8.16 Hz, 4H), 7.85-7.94 (m, 4H), 7.76-7.84 (m, 3H), 7.62-7.72 (m, 3H), 7.59 (dd, *J* = 4.89, 8.91 Hz, 2H), 4.88-4.98 (m, 1H), 4.44 (s, 1H), 3.92 (s, 3H), 3.78 (s, 3H), 3.18-3.38 (m, 2H). **(¹H,¹³C)-HSQC NMR** (400 MHz, DMSO-*d*₆): δ 132.1 (Ar), 129.0 (Ar), 128.7 (Ar), 125.9 (Ar), 126.0 (Ar), 119.7 (Ar), 119.2 (Ar), 115.0 (Ar), 110.6 (Ar), 83.7 (alkyne), 61.0 (OMe), 60.5 (OMe), 54.4 (α-C), 27.7 (β-C); **HRMS (ESI):** *m*/*z* calculated for C₄₄H₃₆N₈O₁₁ [M+H]⁺ 853.2576, found 853.2585 (deviation +1.1 ppm), *t*_{R} = 8.34 min.

### Compound 11:

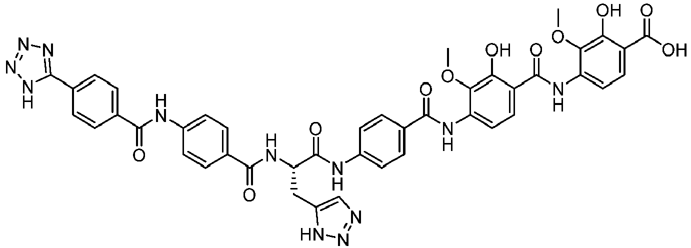

**¹H NMR** (400 MHz, DMSO-*d*₆): δ 11.52 (s, 1H), 11.17 (s, 1H), 10.64 (s, 1H), 10.52 (s, 1H), 9.67 (s, 1H), 8.75 (d, *J=* 7.28 Hz, 1H), 8.16-8.25 (m, 4H), 8.06 (d, *J=* 9.03 Hz, 1H), 7.88-8.01 (m, 6H), 7.75-7.85 (m, 3H), 7.70 (s, 1H), 7.60 (d, *J* = 2.51 Hz, 1H), 7.58 (d, *J* = 2.51 Hz, 1H), 4.88-4.98 (m, *J* = 6.02 Hz, 1H), 3.92 (s, 3H), 3.78 (s, 3H), 3.21-3.36 (m, 2H). **(¹H,¹³C)-HSQC NMR** (400 MHz, DMSO-*d*₆): δ 129.2 (Ar), 129.1 (Ar), 128.7 (Ar), 127.2 (Ar), 126.2 (Ar), 126.0 (Ar), 119.9 (Ar), 119.2 (Ar), 115.1 (Ar), 110.6 (Ar), 60.9 (OMe), 60.5 (OMe), 54.5 (α-C), 27.7 (β-C); **HRMS (ESI):** *m*/*z* calculated for C₄₃H₃₆N₁₂O₁₁ [M+H]⁺ 897.2699, found 897.2725 (deviation +2.9 ppm), *t*_{R} = 7.35 min.

D-E-Isostere 16 was synthesized according to the following synthesis route:

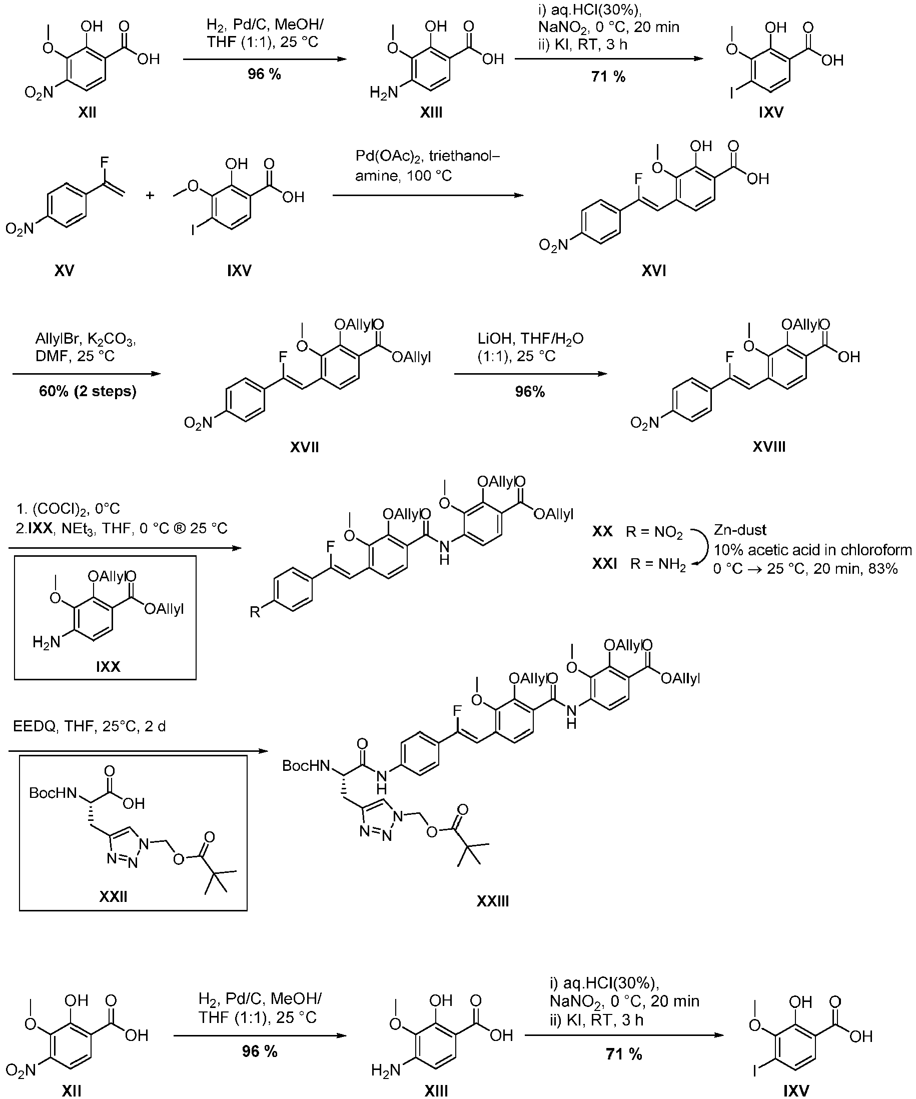

### Preparation of compound XIII:

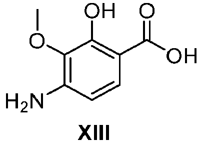

The nitro aromat **XII** (17.0 g, 79.8 mmol, 1.0 eq.) was dissolved in MeOH/THF (1:1, 300 mL) and Pd/C (1.7 g, 10 wt% of nitro aromat **XII**) was added. The mixture was stirred under H2 atmosphere for 2 d. The reaction mixture was then filtrated through Celite and filter cake was washed with ethyl acetate. The filtrate was concentrated reduced pressure to obtain the pruduct **XII** (13.4 g, 96 %) without further purification.
**¹H NMR** (500 MHz, DMSO-*d*₆) δ 11.6 (s, 1H), 7.3 (d, *J* = 8.7 Hz, 1H), 6.2 (d, *J* = 8.7 Hz, 1H), 5.8 (s, 2H), 3.7 (s, 3H);
**¹³C NMR** (126 MHz, DMSO-*d*₆) δ 172.9, 156.0, 148.5, 132.2, 126.6, 106.3, 101.6, 59.4; **HRMS (ESI):** *m*/*z* calc. for C₃H₉NO₄ [M+H]⁺: 184.0604 ; found 184.0612.

### Preparation of aryl iodide IXV

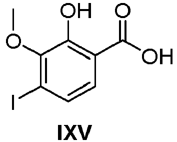

The mixture of the aniline (13.4 g, 73.0 mmol, 1.0 eq.), and 6 N aq. HCI (20 mL) in acetonitrile (80 mL) was cooled to -20 °C (EtOH/ice/N2-liquide). A solution of NaNO₂ (12.6 g, 183 mmol, 2.5 eq.) in H₂O (300 mL) was added dropwise and stirred for 20 min while doing the 2-Naphthol test. Then, a solution of KI (30.3 g, 183 mmol, 2.0 equiv) in H₂O (350 mL) was added dropwise to the solution. After addition, the dark red solid was started to precipitate and the mixture was allowed to warm to room temperature and stirred for additional 2 h. To the solution was added Na₂SO₃. The Solvent was removed under reduced pressure. The resulting solid was filtered, washed with 1N aq. HCl solution, and dried to obtain the product **IXV** (15.3 g, 71 %) as a red-brown solid.
**¹H NMR** (500 MHz, DMSO-*d*₆) δ 7.33 (q, *J* = 8.5 Hz, 2H), 3.78 (s, 3H);
**¹³C NMR** (126 MHz, DMSO-*d*₆) δ 172.4, 154.7, 148.5, 128.3, 126.8, 115.1, 100.9, 60.2; **HRMS (ESI):** *m*/*z* calc. for C₈H₇IO₄ [M+H]⁺: 294.9462 ; found 294.9453.

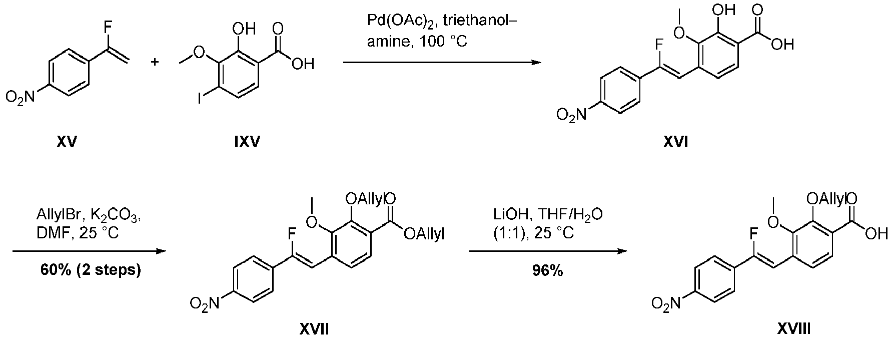

### Preparation of Compound XVI

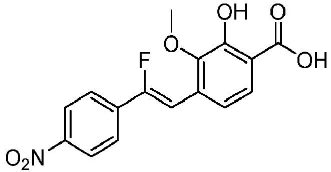

A mixture of the literature known styrene **XV** (1.90 g, 11.4 mmol, 1.00 eq.), the halogenated benzoic acid **IXV** (3.41 g, 11.6 mmol, 1.02 eq.), triethanolamine (10 mL) and Pd(II)acetate (0.26 g, 1.14 mmol, 0.10 eq.) was stirred under argon at 100°C for 24 h. The reaction was cooled to 25°C, quenched by the addition of dil. aq. hydrochloric acid (2 N, 30 mL) and diluted with EtOAc (300 mL). The resulting mixture was filtrated through Celite^{®} and the filter cake washed with ethyl acetate. The organic phase was washed with 1 N HCI (2 x) and Brine (1x), dried over Na₂SO₄ and evaporated. The crude product **XVI** was directly allyl protected without further purification.

### Preparation of Compound XVII

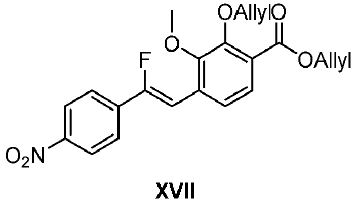

The benzoic acid **XVI** was dissolved in DMF (dry, 20 mL), potassium iodide (0.19 g, 1.14 mmol, 0.10 eq.) and potassium carbonate (6.29 g, 45.5 mmol, 4.00 eq.) were added. The mixture was cooled to 0 °C and allyl bromide was added dropwise. After 16 h at 25 °C the reaction was quenched with water (10 mL) and extracted with Et₂O (4 x 50 mL). The combined org. layers were dried over Na₂SO₄, filtered, and evaporated under reduced pressure. The crude product was purified by flash chromatography (1% → 6% ethyl acetate in cyclohexane) to obtain **XVII** (2.81 g, 6.80 mmol, 60%) as bright yellow solid.
**¹H NMR** (500 MHz, DMSO-*d*₆) δ 8.32 (d, *J* = 8.9 Hz, 2H), 8.05 (d, *J* = 8.9 Hz, 2H), 7.70 (d, *J* = 8.3 Hz, 1H), 7.54 (s, 1H), 7.08 (d, *J* = 40.3 Hz, 1H), 6.10 - 6.00 (m, 2H), 5.44 - 5.35 (m, 2H), 5.26 (ddq, *J* = 26.6, 10.4, 1.4 Hz, 2H), 4.79 (d, *J* = 5.6 Hz, 2H), 4.54 (d, *J* = 5.8 Hz, 2H), 3.87 (d, *J* = 5.5 Hz, 3H).
**¹⁹F NMR** (471 MHz, DMSO) δ -112.99, -113.08.
**¹³C NMR** (126 MHz, DMSO-*d*₆) δ 164.65, 151.72, 151.07, 147.74, 133.94, 132.42, 127.78, 125.73, 125.67, 125.37, 124.13, 118.31, 117.79, 102.92, 74.57, 65.38, 61.49.
**HRMS (ESI):** *m*/*z* calc. for C₂₂H₂₀FNO₆ [M+H]⁺: 414.1347; found 414.1349.

### Preparation of Compound XVIII

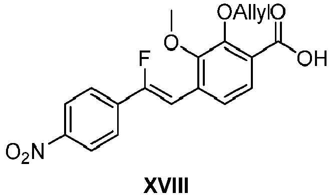

Allylester **XVIII** (1.80 g, 4.35 mmol, 1.00 eq) was dissolved in THF (20 mL). A solution of LiOH (12.00 eq., 52.3 mmol, 1.25 g) in water (20 mL) was added. After 16 h at 25 °C the reaction mixture was concentrated *in vacuo* and diluted with H₂O (10 mL). The product was precipitated with 6 N HCl, filtered and washed with H₂O. Compound **XVIII** was obtained as a light yellow solid (1.57 g, 4.21 mmol, 96 %).
**¹H NMR** (500 MHz, DMSO-*d*₆) δ 8.31 (d, *J* = 8.9 Hz, 2H), 8.04 (d, *J* = 8.9 Hz, 2H), 7.65 (d, *J* = 8.3 Hz, 1 H), 7.48 (d, *J* = 8.3 Hz, 1 H), 7.07 (d, *J* = 40.4 Hz, 1H), 6.08 (ddt, *J* = 17.3, 10.8, 5.6 Hz, 1H), 5.38 (dq, *J* = 17.2, 1.7 Hz, 1 H), 5.22 (dt, *J* = 10.4, 1.5 Hz, 1H), 4.53 (dd, *J* = 5.6, 1.7 Hz, 2H), 3.86 (s, 3H).
**¹⁹F NMR** (471 MHz, DMSO) δ -113.48, -113.57.
**¹³C NMR** (126 MHz, DMSO) δ 167.08, 152.12, 151.41, 148.16, 134.65, 130.47, 128.02, 126.16, 126.10, 125.79, 124.90, 124.63, 118.01, 103.53, 103.47, 74.98, 61.96, 40.54, 40.45, 40.37, 40.28, 40.20, 40.11, 39.94, 39.78, 39.61, 39.44.
**HRMS (ESI):** *m*/*z* calc. for C₁₉H₁₆FNO₆ [M+H]⁺: 374.1034 ; found 374.1032.

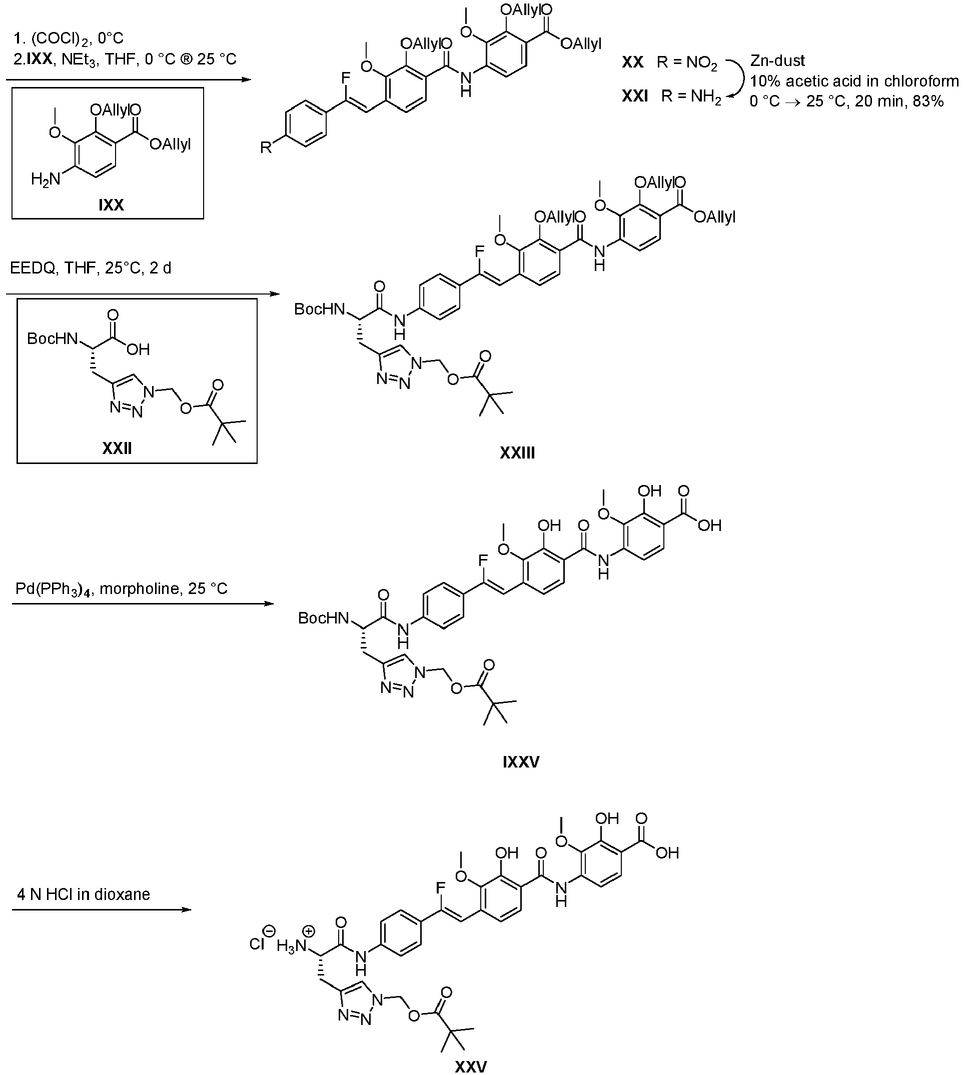

### Preparation of Compound XX

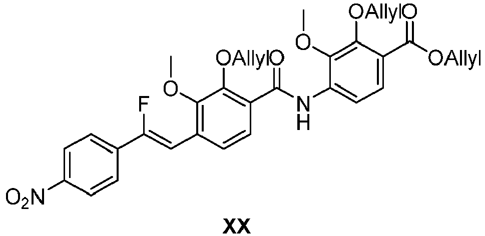

To a solution of compound **XVIII** (272 mg, 0.72 mmol, 1.00 eq.) in CH₂Cl₂ (dry, 10 mL) was added oxalyl chloride (0.19 mL, 2.20 mmol, 3.00 eq.) and a catalytic amount of DMF (50 µL) at 0 °C. The mixture was stirred 3 h at 25 °C. The solution was concentrated under reduced pressure and the residue dissolved in CH₂Cₗ₂ (10 mL). Then literature known amine **IXX** (211 mg, 0.80 mmol, 1.10 eq.) and triethylamine (0.254 mL, 1.82 mmol, 2.50 eq.) were added. The mixture was stirred for 12 h at 25 °C shielded from light and then quenched with water (15 mL). The aqueous phase was extracted with CH2CI2 (3 x 50 mL), the combined organic phases were washed with 2 N HCI (aq.), water, saturated NaHCO₃ and brine, dried over Na₂SO₄, filtered and evaporated under reduced pressure. The residue was purified by column chromatography on silica gel (cyclohexane: ethyl acetate, 50: 1 → 25:1 →10:1) to obtain **XX** (196 mg, 0.32 mmol, 44%) as light yellow solid.
**¹H NMR** (DMSO-d₆, 400 MHz): δ 10.67 (s, 1H), 8.33 (dd, *J* = 10.4, 8.3 Hz, 3H), 8.08 (d, *J* = 9.0 Hz, 2H), 7.87 - 7.76 (m, 2H), 7.57 (d, *J* = 8.8 Hz, 1H), 7.13 (d, *J* = 40.3 Hz, 1H), 6.18 - 5.97 (m, 4H), 5.40 (ddt, *J* = 17.2, 11.7, 1.7 Hz, 4H), 5.27 (ddt, *J* = 14.9, 10.3, 1.5 Hz, 4H), 4.83 - 4.74 (m, 4H), 4.54 (dt, *J* = 5.7, 1.4 Hz, 2H), 3.93 (dd, *J* = 2.3, 0.6 Hz, 6H).
**¹⁹F NMR** (471 MHz, DMSO) δ -112.65, -112.73.
**¹³C NMR** aus HSQC_ed(101 MHz, DMSO) δ 124.45, 115.07, 125.92, 125.80, 125.92, 103.17, 133.11, 119.33, 75.37, 65.61, 74.83, 61.91.
**HRMS (ESI):** *m*/*z* calc. for C₃₃H₃₁FN₂O₉ [M+H]⁺: 619.2086; found 619.2083.

### Preparation of Compound XXI

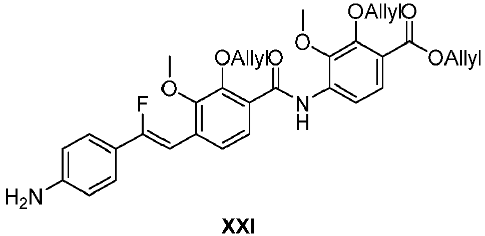

Compound **XX** (480 mg, 0.78 mmol, 1.00 eq.) was dissolved in a mixture of chloroform (45 ml) and acetic acid (5 ml) and cooled to 0 °C. Zinc dust (1.01 g, 15.5 mmol, 20 eq.) was added portion wise. After 20 min the reaction was proven to be complete (verified by TLC-control). The solid was filtered and washed with CH2CI2 (3 x 50 ml). The combined liquids were evaporated to dryness. The residue was taken up in CH2CI2 (100 ml) and saturated aqueous NaHCO₃-Solution (300 ml). The aqueous phase was further extracted twice with CH2CI2 (2 x 50 ml). The combined organic fractions were washed successively with saturated aqueous NaHCO₃-Solution (1 x 100 ml), distilled water (1 x 100 ml) and brine (1 x 100 ml), dried over Na₂SO₄ and evaporated to obtain **XXI** (420 mg, 0.71 mmol, 92%) as a yellow solid.
**¹H NMR** (500 MHz, DMSO-*d*₆) δ 10.69 (s, 1H), 8.33 (d, *J* = 8.8 Hz, 1H), 7.79 (d, *J* = 8.6 Hz, 1H), 7.71 (d, *J* = 8.5 Hz, 1H), 7.57 (d, *J* = 8.8 Hz, 1H), 7.44 (d, *J* = 8.7 Hz, 2H), 6.64 (d, *J* = 8.6 Hz, 2H), 6.46 (d, *J* = 41.9 Hz, 1H), 6.14 - 6.03 (m, 3H), 5.73 (s, 2H), 5.43 - 5.37 (m, 3H), 5.29 - 5.20 (m, 3H), 4.80 - 4.76 (m, 4H), 4.54 (dt, J = 5.7, 1.5 Hz, 2H), 3.92 (s, 3H), 3.88 (s, 3H).
**¹⁹F NMR** (471 MHz, DMSO) δ -109.92, -110.01.
**¹³C NMR** (126 MHz, DMSO) δ 164.43, 162.46, 151.06, 150.86, 149.74, 142.46, 136.51, 134.67, 133.94, 133.17, 132.84, 132.61, 127.51, 126.26, 126.25, 126.03, 125.97, 125.63, 120.27, 119.91, 118.11, 117.82, 114.78, 113.57, 79.15, 75.13, 74.53, 65.07, 61.18, 60.99.
**HRMS (ESI):** *m*/*z* calc. for C₃₃H₃₃FN₂O₇ [M+H]⁺: 589.2345 ; found 589.2348.

### Preparation of XXIII

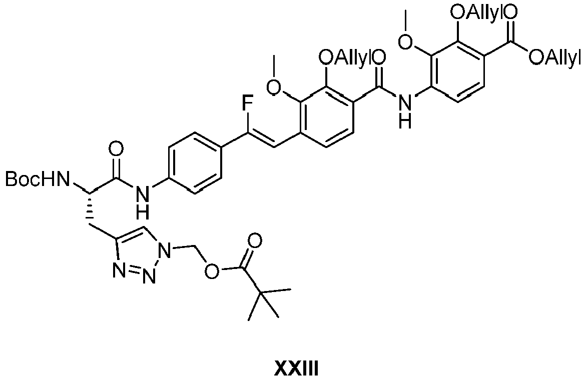

Literature known Boc-b-(1-pivaloyloxymethyl)-1,2,3-triazol-4-yl)-Alanine (528 mg, 1.43 mmol, 2.00 eq.) was dissolved in THF (10 ml) and cooled to 0 °C. N-Ethoxycarbonyl-2-ethoxy-1,2-dihydroqinoline (EEDQ) (1.49 g, 6.01 mmol, 3.00 eq) was added and after 5 minutes compound **XXI** (420 mg, 0.71 mmol, 1.00 eq.) was added. The reaction mixture was slowly warmed to room temperature and stirred for 32 h shielded from light. All volatiles were removed *in vacuo* and the residue was taken up in ethyl acetate (100 ml). The organic fraction was washed with saturated aqueous NaHCO₃-Solution (3 x 50 ml) and brine (1 x 50 ml), dried over Na₂SO₄ and evaporated. The residue was purified *via* flash chromatography on silica gel (cyclohexane: ethyl acetate; 4:1→3:1→2:1→3:2) Compound **XXIII** (598 mg, 0.635 mmol, 89 %) was obtained as a colorless solid.
**¹H NMR** (500 MHz, DMSO-*d*₆) δ 10.67 (s, 1H), 10.35 (s, 1H), 8.32 (d, *J* = 8.7 Hz, 1H), 7.97 (s, 1H), 7.81 (d, *J* = 8.5 Hz, 1H), 7.75 (d, *J* = 11.7 Hz, 5H), 7.57 (d, *J* = 8.7 Hz, 1H), 7.18 (d, *J* = 8.0 Hz, 1H), 6.74 (d, *J* = 41.1 Hz, 1H), 6.28 (s, 2H), 6.17 - 5.98 (m, 3H), 5.40 (dddd, *J* = 17.2, 14.8, 3.5, 1.7 Hz, 3H), 5.30 - 5.23 (m, 3H), 4.82 - 4.76 (m, 4H), 4.54 (dt, *J* = 5.7, 1.4 Hz, 2H), 4.40 (q, *J* = 8.2, 7.7 Hz, 1H), 3.92 (d, *J* = 11.4 Hz, 7H), 3.12 (dd, *J* = 14.8, 5.1 Hz, 1H), 3.00 (dd, *J* = 14.7, 9.4 Hz, 1H), 1.38 (d, *J=* 17.2 Hz, 9H), 1.09 (s, 9H).
**¹⁹F NMR** (471 MHz, DMSO) δ -111.13, -111.22.
**¹³C NMR** from HSQC_ed (126 MHz, DMSO) δ 115.28, 124.41, 125.84, 125.71, 119.80, 126.70, 126.74, 97.80, 70.12, 133.86, 118.67, 119.11, 133.96, 119.38, 119.69, 75.67, 65.53, 75.00, 55.38, 61.50, 61.85, 63.57, 28.32, 28.63, 26.96.
**HRMS (ESI):** m/z calc. for C₄₉H₅₇FN₆O₁₂ [M+H]⁺: 941.4091; found 941.4092.

### Preparation of compound IXXV:

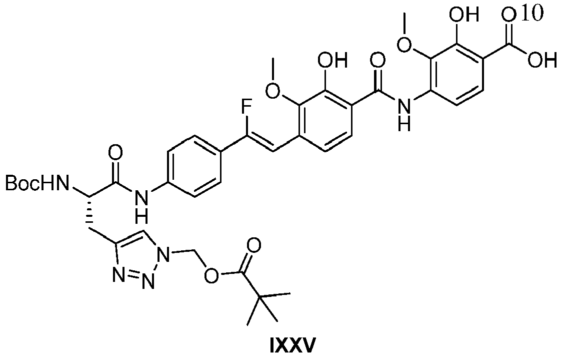

Tetrapeptide **XXIII** (378 mg, 0.401 mmol, 1.00 eq.) was dissolved in THF (5 ml), morpholine (0.70 mL, 8.03 mmol, 20.0 eq.) and *tetrakis*(triphenylphosphin)palladium (0) (186 mg, 0.160 mmol, 0.40 eq.) were added. The mixture was stirred for 2.5 h shielded from light. All volatiles were removed *in vacuo* and the residue was purified via column chromatography (2%→10% MeOH, 2% steps in CH2CI2). Compound **IXXV** (290 mg, 0.353 mmol, 88%) was obtained as a white solid.
**¹H NMR** (DMSO-d₆, 500MHz): δ 11.40 (s, 1H), 10.93 (s, 1H), 10.35 (s, 1H), 7.97 (s, 1H), 7.82 (d, *J* = 8.6 Hz, 1H), 7.74 (s, 5H), 7.71 (d, *J* = 7.7 Hz, 4H), 7.45 (dd, *J* = 8.6, 5.6 Hz, 3H), 7.18 (d, *J* = 8.1 Hz, 1H), 6.69 (d, *J* = 41.2 Hz, 1H), 6.28 (s, 3H), 4.40 (dd, *J* = 14.5, 7.8 Hz, 1H), 3.87 (s, 4H), 3.79 (s, 4H), 3.11 (d, *J* = 12.0 Hz, 1H), 3.00 (dd, *J* = 14.7, 9.4 Hz, 1H), 1.36 (s, 10H), 1.09 (s, 13H).
**¹⁹F NMR** (471 MHz, DMSO) δ -111.31, -111.40.
**¹³C NMR** from HSQC (DMSO-d₆, 126MHz): δ 124.40, 125.83, 125.74, 119.80, 108.31, 125.31, 119.94, 98.20, 70.12, 55.49, 60.04, 61.91, 70.36, 28.35, 28.63, 26.95.
**HRMS** (ESI): m/z calc. for C₄₀H₄₅FN₆O₁₂ [M+H]⁺: 821.3152, found 821.3149.

### Preparation of compound XXV:

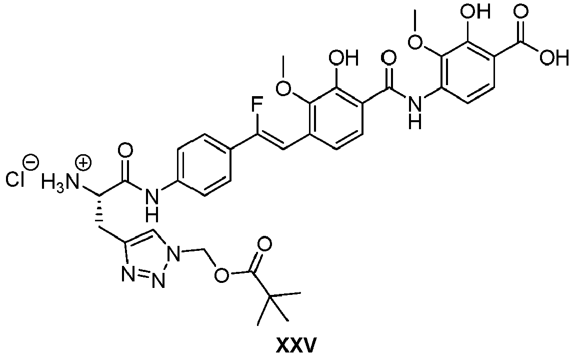

Tetrapeptide **IXXV** (120 mg, 0.146 mmol, 1.00 eq.) was dissolved in 4 N HCI in dioxane and stirred for 1 hour. The solvent was evaporated *in vacuo* and the product **XXV** (101 mg, 0.133 mmol, 91%) was obtained as white solid. Compound **XXV** was used in the next step without further characterization.
**HRMS** (ESI): m/z calc. for C₃₅H₃₇FN₆O₁₀ [M+H]⁺: 721.2628, found: 721.2623.

### Preparation of active ester XI

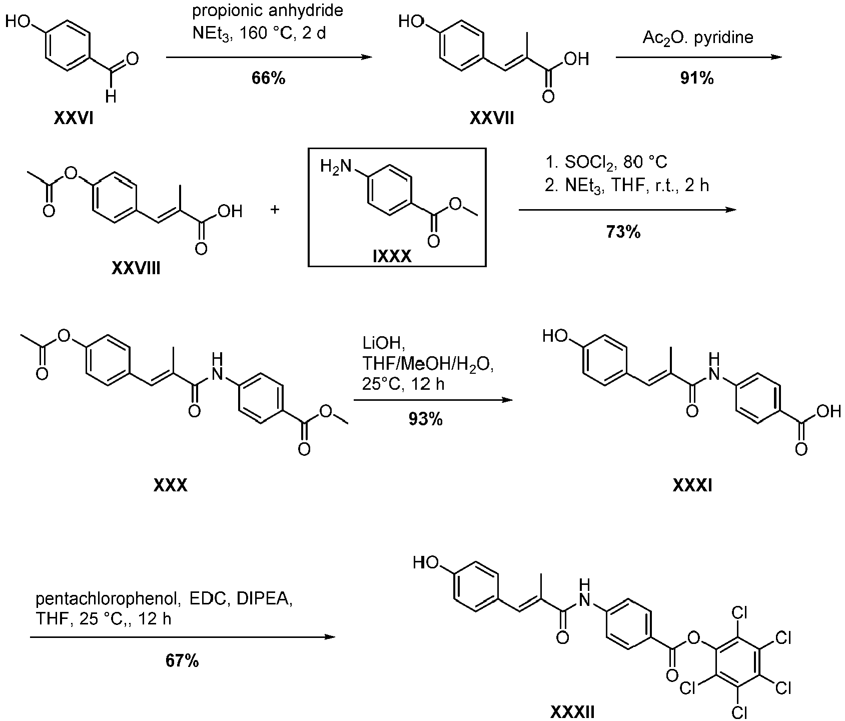

### Preparation of compound XXVI

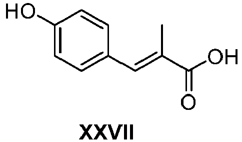

To a solution of commercially available benzaldehyde **XXVI** (1.0 eq, 205 mmol, 25.0 g) in propionic anhydride (209 mL, 1.64 mol, 8.00 eq.) was added NEt₃ (200 mL, 1.43 mmol, 7.00 eq.). The reaction mixture was refluxed for 2 d at 160 °C. Then conc. H₂SO₄ (60 mL) diluted with H₂O (300 mL) was added slowly at 0 °C. The yellow precipitate was filtered and dried *in vacuo.* The precipitate was dissolved in a THF/MeOH (200 mL) mixture and 3 N KOH_{aq.} (100 mL) was added. After stirring 12 h at 25 °C the reaction mixture was filtered again, the filtrate was diluted with saturated K₂CO₃ (100 mL), the aqueous layer washed with ethyl acetate (4 x 100 mL) and then acidified with 6 N HCl_{aq.} (200 mL) to pH 1. The precipitate was filtered and dried in vacuo to obtain **XXVII** (24.1 g, 135 mmol, 66%) as colourless solid.
**¹H NMR** (DMSO-d₆ ,500MHz): **d** = 12.25 (s, 1H), 9.80 (s, 1H), 7.51 (s, 1H), 7.34 (d, *J* = 8.7 Hz, 2H), 6.82 (d, *J* = 8.7 Hz, 2H), 2.02 (d, *J* = 1.5 Hz, 3H).
**¹³C NMR** (DMSO-d₆ ,126MHz): **d** = 169.67, 157.90, 137.88, 131.61, 126.38, 125.02, 115.39, 13.93.
**HRMS** (ESI): m/z calc. for C₁₀H₁₀O₃ [M-H]⁻: 177.0557, found: 177.0561.

### Preparation of compound XXVIII

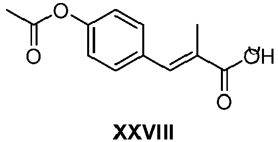

To a suspension of compound **XXVII** (6.00 g, 168 mmol, 1.00 eq.) in pyridine (10.85 mL, 135 mmol, 4.0 eq.) was added acetic anhydride (15.9 mL, 168 mmol, 5.00 eq.). The reaction mixture was stirred at 100 °C for 12 h. All volatiles were removed *in vacuo* and the resulting oil was diluted with 1 N HCl_{aq.} The precipitate was filtered and dried *in vacuo* to obtain **XXVIII** (6.75 g, 30.6 mmol, 91%) as colorless solid.
**¹H NMR** (400 MHz, DMSO-*d*₆) δ 12.49 (s, 1H), 7.59 (d, *J* = 1.7 Hz, 1H), 7.52 (d, *J* = 8.5 Hz, 2H), 7.19 (d, *J* = 8.6 Hz, 2H), 2.28 (s, 3H), 2.03 (d, *J* = 1.5 Hz, 3H).
**¹³C NMR** (101 MHz, DMSO) δ 169.31, 169.11, 150.31, 136.75, 133.11, 130.84, 128.69, 122.15, 121.95, 20.87, 13.90.
**HRMS** (ESI): m/z calc. for C₁₂H₁₂O₄ [M-H]⁻: 219.0663, found: 219.0659.

### Preparation of Compound XXX

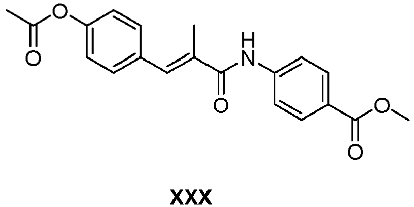

Compound **XXVIII** (4.33 g, 18.1 mmol, 1.00 eq.) was dissolved in thionyl chloride (20 mL), a catalytic amount of DMF (100 µL) was added. The mixture was stirred 2 h at 120 °C. The solution was concentrated under reduced pressure and the residue dissolved in THF (20 mL). Then literature known amine **IXXX** (2.97 g, 19.7 mmol, 1.00 eq.) and triethylamine (7.42 mL, 54.4 mmol, 3 eq.) in THF (10 mL) were added to the above solution. The mixture was stirred for 12 h at 25 °C shielded from light. All volatiles were removed *in vacuo.* The residue was dissolved in CH₂Cl₂ and washed with 2 N HCI (aq.), water, saturated NaHCO₃ and brine, dried over Na₂SO₄, filtered, and evaporated under reduced pressure to obtain **XXX** (5.10 g, 14.4 mmol, 73%) as colorless solid.
**¹H NMR** (DMSO-d₆ ,500MHz): **d** = 10.26 (s, 1H), 7.94 (d, *J* = 8.8 Hz, 2H), 7.88 (d, *J* = 8.9 Hz, 2H), 7.53 (d, *J* = 8.5 Hz, 2H), 7.34 (s, 1H), 7.22 (d, *J* = 8.6 Hz, 2H), 3.83 (s, 3H), 2.29 (s, 3H), 2.13 (d, *J* = 1.4 Hz, 3H).f
**¹³C NMR** (DMSO-d₆ ,126MHz): **d** = 169.09, 168.63, 165.84, 150.04, 143.78, 133.23, 132.84, 130.57, 130.05, 124.01, 121.92, 119.37, 51.85, 20.86, 14.43.
**HRMS** (ESI): m/z calc. for C₂₀H₁₉NO₅ [M+H]⁺: 354.1336, found: 354.1332.

### Preparation of compound XXXI

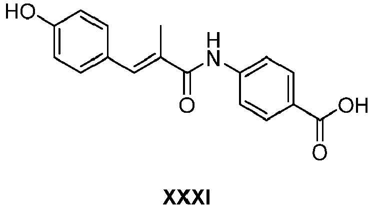

Methylester **XXX** (5.00 g, 14.2 mmol, 1.00 eq.) was dissolved in a THF/MeOH (40 mL) mixture. A solution of LiOH (1.69 g, 70.8 mmol, 5.00 eq.) in water (20 mL) was added. After 12 h at 25 °C the reaction mixture was concentrated *in vacuo* and diluted with H₂O (10 mL). The product was precipitated with 6 N HCl, filtered and washed with H₂O. Compound **XXXI** (3.91 g, 14.5 mmol, 93 %) was obtained as a light yellow solid.
**¹H NMR** (DMSO-d₆ ,500MHz): **d** = 12.66 (s, 1H), 10.17 (s, 1H), 9.82 (s, 1H), 7.91 (d, *J* = 8.9 Hz, 2H), 7.86 (d, *J* = 8.9 Hz, 2H), 7.35 (d, *J* = 8.7 Hz, 2H), 7.28 (s, 1H), 6.86 (d, *J* = 8.6 Hz, 2H), 2.11 (d, *J* = 1.5 Hz, 3H).
**¹³C NMR** (DMSO-d₆ ,126MHz): **d** = n.d.
**HRMS** (ESI): m/z calc. for C₁₇H₁₅NO₄ [M+H]⁺: 298.1074, found: 298.1071.

### Preparation of compound XXXII

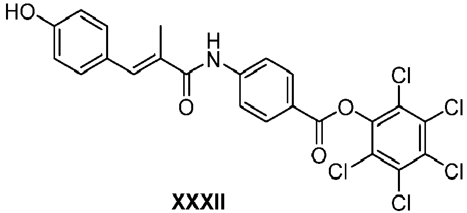

Compound **XXXI** (5.00 g, 16.8 mmol, 1.00 eq.), EDC*HCl (3.92 mmol, 25.2 mmol, 1.50 eq.) and DIPEA (7.40 mL, 42.6 mmol, 2.53 eq.) were dissolved in THF (30 mL). After 1 min at 25 °C, pentachlorophenol (4.70 g, 17.7 mmol, 1.05 eq.) was added and resulting the reaction mixture was stirred for another 16 h at 25 °C Afterwards, the solution was dissolved with EtOAc (50 mL) and washed with H₂O (2 x 30 mL), 10 % KHSO₄ (2 x 30 mL) and brine (1 x 30 mL). All volatiles were removed *in vacuo* and the reaction mixture was diluted with cold Et₂O/hexane (4:1, 30 mL), the formed precipitate was filtered, and washed with Et₂O. Active ester **XXXII** was obtained as a colorless solid (6.21 g, 11.4 mmol, 67 %).
**¹H NMR** (400 MHz, DMSO-*d*₆) δ 10.37 (s, 1H), 9.81 (s, 1H), 8.17 (d, *J* = 8.8 Hz, 2H), 8.01 (d, *J* = 8.8 Hz, 2H), 7.37 (d, *J* = 8.7 Hz, 2H), 7.31 (s, 1H), 6.85 (d, *J* = 8.6 Hz, 2H), 2.13 (d, *J* = 1.3 Hz, 3H).
**¹³C NMR** (DMSO-d₆ ,126MHz): **d** = n.d.
**HRMS** (ESI): m/z calc. for C₂₃H₁₄Cl₅NO₄ [M+H]⁺: 545.9409, found: 545.9392.

### Preparation of compound 16:

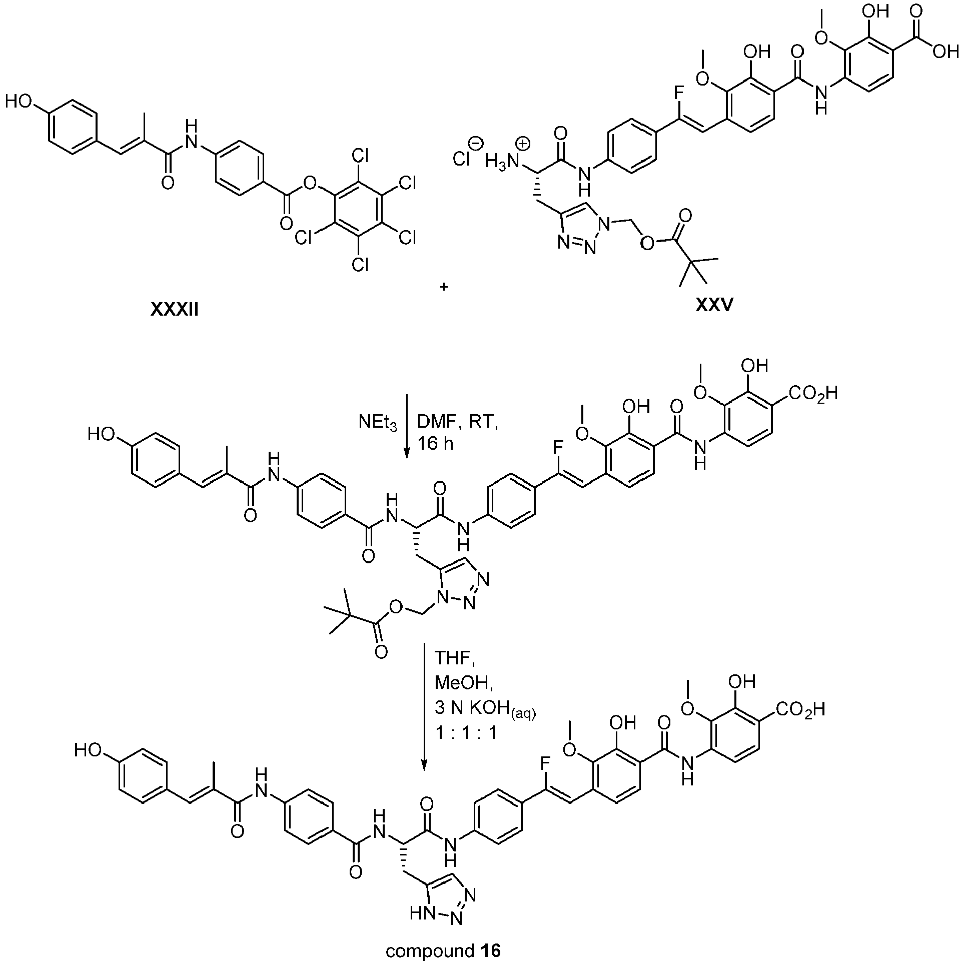

Compound **XXV** (110 mg, 0.145 mmol, 1.00 eq) was dissolved in DMF (2 ml) and triethylamine (0.20 mL, 1.45 mmol, 10.0 eq.) was added. After adding the active ester (95.1 mg, 0.174 mmol, 1.20 eq.), the mixture was stirred for 32 h shielded from light. All volatiles were removed *in vacuo.* Then cooled to 0 °C and 3 N KOH_{(aq)} (1 ml) was added dropwise. After 15 min of stirring, 550 µl of 6 N HCl_{(aq)} were added dropwise. The resulting mixture was evaporated to dryness. The residue was purified *via* prep HPLC. Compound **2** (21 mg, 0.021 mmol, 14%) was obtained as a white fluffy solid.

### Analytical Data for compound 16:

**¹H NMR** (700 MHz, DMSO-*d*₆): δ 11.58 (s, 1H), 11.41 (s, 1H), 11.24 (s, 1H), 10.47 (s, 1H), 10.08 (s, 1H), 9.77 (s, 1H), 8.68 (d, *J* = 7.7 Hz, 1H), 8.08 (d, *J* = 8.8 Hz, 1H), 7.88 - 7.85 (m, 2H), 7.85 - 7.73 (m, 7H), 7.68 (s, 1H), 7.60 (d, *J* = 8.9 Hz, 1H), 7.48 (d, *J* = 8.3 Hz, 1H), 7.35 (d, *J* = 8.7 Hz, 2H), 7.26 (s, 1H), 6.84 (d, *J* = 8.4 Hz, 2H), 6.70 (d, *J* = 41.2 Hz, 1H), 4.90 (q, *J* = 7.8, 7.4 Hz, 1H), 3.92 (s, 3H), 3.80 (s, 3H), 3.29 (dd, *J* = 14.7, 5.6 Hz, 1H), 3.23 (dd, *J* = 14.8, 9.1 Hz, 1H), 2.11 (s, 3H).
**(¹H,¹³C)-HSQC NMR** (176 MHz, DMSO) δ 110.56, 128.68, 119.54, 126.20, 128.74, 119.52, 119.83, 125.81, 126.19, 120.28, 131.78, 134.38, 115.84, 98.13, 98.10, 54.64, 60.66, 62.05, 27.80, 15.02.
**HRMS (ESI):** *m*/*z* [M+H]⁺calcd. for C₄₆H₄₀FN₇O₁₁: 886.2843, found 886.2854, *t*_{R} = 8.64 min.

The following compounds are obtained in an analog synthesis procedures.

### Compound 14:

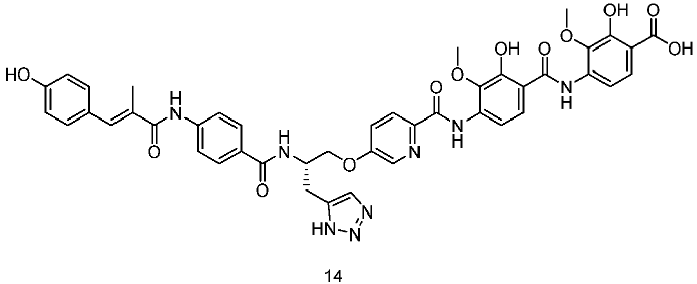

**¹H NMR** (700 MHz, DMSO-*d*₆) δ 11.71 (s, 1H), 11.12 (s, 1H), 10.44 (s, 1H), 10.05 (s, 1H), 9.76 (s, 1H), 8.48 (d, *J* = 2.8 Hz, 1H), 8.44 (d, *J* = 8.1 Hz, 1H), 8.17 (d, *J* = 8.7 Hz, 1H), 8.11 (d, *J* = 8.9 Hz, 1H), 8.02 (q, *J* = 9.2, 8.1 Hz, 1H), 7.87 (d, *J* = 8.8 Hz, 1H), 7.79 (s, 4H), 7.71 (dd, *J* = 8.7, 2.9 Hz, 1H), 7.59 (d, *J* = 8.8 Hz, 1H), 7.34 (d, *J* = 8.3 Hz, 2H), 7.25 (s, 1H), 6.83 (d, *J* = 8.3 Hz, 2H), 4.64 (h, *J* = 6.7, 6.2 Hz, 1H), 4.34 (dd, *J* = 10.1, 6.2 Hz, 1H), 4.30 (dd, *J* = 10.1, 5.4 Hz, 1H), 3.91 (s, 3H), 3.85 (s, 3H), 3.17 (dd, *J* = 14.8, 5.7 Hz, 1H), 3.10 (dd, *J* = 14.9, 8.4 Hz, 1H), 2.10 (s, 3H); **(¹H,¹³C)-HSQC** (176 MHz, DMSO) δ 137.19, 123.63, 110.12, 110.37, 126.42, 118.94, 127.89, 122.28, 125.62, 131.24, 133.79, 115.30, 48.49, 69.67, 60.09, 60.72, 26.48, 14.44; **HRMS (ESI):** *m*/*z* [M+H]⁺*calcd.* for C₄₄H₄₀N₈O₁₂: 873.2838, found 873.2842, *t*_{R} = 8.21 min.

### Compound 15:

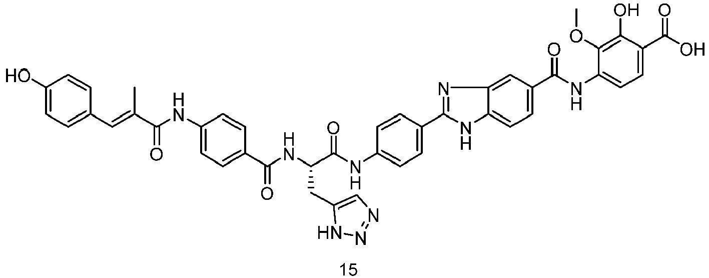

**¹H NMR** (500 MHz, DMSO-*d*₆): δ 10.58 (s, 1H), 10.11 (s, 1H), 9.78 (s, 1H), 9.61 (s, 1H), 8.72 (d, *J* = 7.28 Hz, 1H), 8.16-8.25 (m, 3H), 7.85-7.92 (m, 5H), 7.82 (d, *J* = 7.28, 2H) 7.77 (d, *J* = 7.28, 1H), 7.68-7.72 (m, 2H), 7.60 (d, *J* = 8.9 Hz, 1H), 7.36 (d, *J* = 8.9 Hz, 2H), 7.27 (s, 1H), 6.85 (d, *J* = 8.4 Hz, 2H), 4.95-4.90 (m, 1H), 3.92 (s, 3H), 3.31-3.27 (m, 1H), 3.28-3.26 (m, 1H), 2.12 (s, 3H). **(¹H,¹³C)-HSQC NMR** (126 MHz, DMSO-*d*₆): δ 128.20, 119.79, 128.62, 119.57, 128.58, 112.87, 125.35, 131.76, 134.41, 115.88, 54.79, 60.60, 46.02, 15.02; **HRMS (ESI):** *m*/*z* calculated for C₄₄H₃₇N₉O₉ [M+H]⁺ 836.27, found 836.28, *t*_{R} = 6.50 min.

### Compound 17:

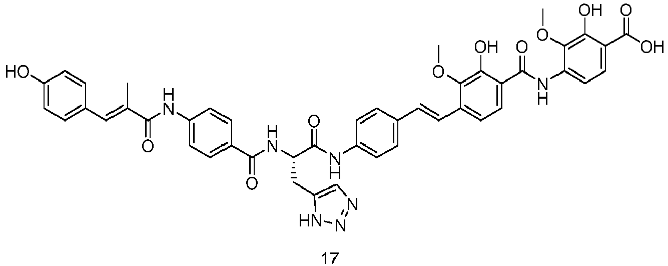

**¹H NMR** (700 MHz, DMSO-*d*₆): ¹H NMR (500 MHz, DMSO-*d*₆) δ 11.59 (s, 1H), 11.38 (s, 1H), 11.23 (s, 1H), 10.32 (s, 1H), 10.07 (s, 1H), 9.75 (s, 1H), 8.65 (d, *J* = 7.8 Hz, 1H), 8.07 (d, *J* = 8.9 Hz, 1H), 7.87 (d, *J* = 8.9 Hz, 2H), 7.81 (dd, *J* = 8.7, 7.1 Hz, 3H), 7.69 (d, *J* = 8.5 Hz, 2H), 7.65 - 7.58 (m, 3H), 7.40 (d, *J* = 8.8 Hz, 1H), 7.39 - 7.33 (m, 3H), 7.33 - 7.26 (m, 2H), 6.85 (d, *J* = 8.7 Hz, 2H), 4.91 (q, *J* = 7.8 Hz, 1H), 3.92 (s, 3H), 3.80 (s, 3H), 3.25 (td, *J* = 14.6, 14.1, 7.3 Hz, 2H), 2.12 (d, *J* = 1.4 Hz, 3H); **(¹H,¹³C)-HSQC** (126 MHz, DMSO) δ 110.64, 128.60, 119.42, 126.03, 119.87, 127.92, 126.03, 115.85, 131.82, 134.40, 115.85, 54.54, 60.64, 61.97, 14.99; **HRMS (ESI):** *m*/*z* [M+H]⁺ calcd. for C₄₆H₄₁N₇O₁₁: 868.2937, found 868.2943, *t*_{R} = 8.43 min.

### Compound 18:

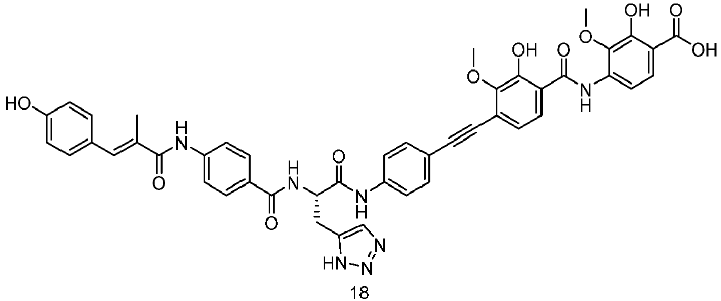

**¹H NMR** (700 MHz, DMSO-*d*₆): ¹H NMR (700 MHz, DMSO-*d*₆) δ 11.59 (s, 1H), 11.55 (s, 1H), 11.26 (s, 1H), 10.45 (s, 1H), 10.07 (s, 1H), 9.77 (s, 1H), 8.68 (d, *J* = 7.6 Hz, 1H), 8.08 (d, *J* = 8.9 Hz, 1H), 7.86 (d, *J* = 8.3 Hz, 2H), 7.79 (dd, *J* = 20.1, 8.1 Hz, 3H), 7.73 (d, *J* = 8.3 Hz, 2H), 7.67 (s, 1H), 7.58 (dd, *J* = 20.2, 8.6 Hz, 3H), 7.35 (d, *J* = 8.1 Hz, 2H), 7.26 (s, 1H), 7.13 (d, *J* = 8.4 Hz, 1H), 6.84 (d, *J* = 8.1 Hz, 2H), 4.89 (q, *J* = 7.6 Hz, 1H), 3.99 (s, 3H), 3.92 (s, 3H), 3.24 - 3.20 (m, 2H), 2.11 (s, 3H); **(¹H,¹³C)-HSQC** ¹³C NMR (176 MHz, DMSO) δ 110.46, 128.66, 128.51, 119.51, 126.02, 119.84, 126.21, 132.65, 131.77, 134.33, 123.50, 115.84, 54.68, 61.57, 60.64, 15.01; **HRMS (ESI):** *m*/*z* [M+H]⁺calcd. for C₄₆H₃₉N₇O₁₁: 866.2780, found 866.2771, *t*_{R} = 8.06 min.

### Compound 18b:

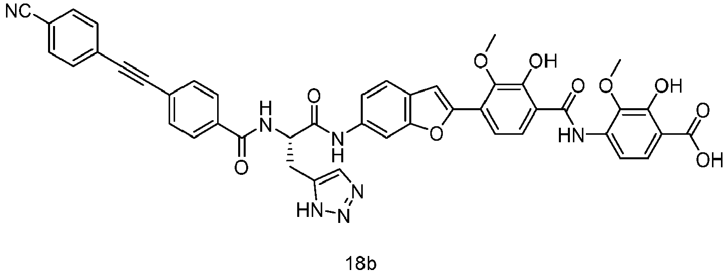

**¹H NMR** (700 MHz, DMSO-*d*₆) δ 14.64 (s, 1H), 11.70 (s, 1H), 11.21 (s, 1H), 10.48 (s, 1H), 9.00 (d, *J* = 7.7 Hz, 1H), 8.16 (s, 1H), 7.97 (d, *J* = 8.2 Hz, 2H), 7.95 - 7.90 (m, 4H), 7.78 (dd, *J* = 8.2, 6.3 Hz, 3H), 7.72 (d, *J* = 8.4 Hz, 2H), 7.69 - 7.64 (m, 2H), 7.60 - 7.54 (m, 3H), 7.43 - 7.40 (m, 1H), 4.96 (d, *J* = 8.5 Hz, 1H), 3.92 (s, 3H), 3.83 (s, 3H), 3.25 (d, *J* = 14.3 Hz, 2H); **(¹H,¹³C)-HSQC** (176 MHz, DMSO) δ 102.25, 128.51, 133.09, 126.80, 132.77, 132.05, 122.07, 132.62, 117.17, 126.08, 107.89, 127.16, 116.39, 54.82, 60.55, 60.24, 28.13, 29.39, 9.06; **HRMS (ESI):** *m*/*z* [M+H]⁺ calcd. for C₄₅H₃₃N₇O₁₀: 832.2362, found 832.2354, *t*_{R} = 9.82 min.

### Compound 32:

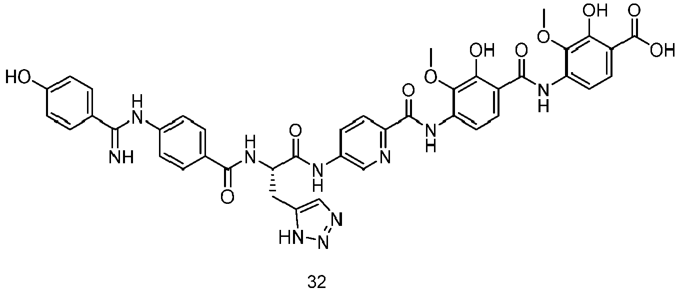

**¹H NMR** (DMSO-d₆ ,500MHz): δ 11.74 (s, 1H), 11.60 (br. s, 1H), 11.13 (s, 1H), 10.88 (s, 1H), 10.49 (s, 1H), 10.00 (s, 1H), 8.99 (d, *J* = 2.0 Hz, 1H), 8.78 (d, *J* =7.45 Hz, 1H), 8.35 (dd, *J* = 8.6, 2.1 Hz, 1H), 8.21 (d, *J* = 8.6 Hz, 1H), 8.12 (d, *J* = 9.3 Hz, 1H), 8.00-7.87 (m, 4H), 7.76 (d, *J* = 8.9 Hz, 2H), 7.70(br. s, 1H), 7.60 (d, *J* = 8.9 Hz, 1H), 6.62 (d, *J* = 8.4 Hz, 2H), 4.94-4.93 (m, 1H), 3.92 (s, 3H), 3.88 (s, 3H), 3.34-3.31 (m, 1H), 3.28-3.26 (m, 1H). **(¹H,¹³C)-HSQC** (176 MHz, DMSO) δ 139.97, 117.02, 127.77, 123.45, 131.80, 110.67, 110.83, 119.89, 128.67, 119.47, 130.10, 111.80, 126.20, 127.98, 113.23, 54.73, 63.80, 57.72, 60.71, 61.21, 64.18, 58.17, 60.52, 60.52, 70.18, 27.61; **HRMS (ESI):** *m*/*z* calculated for C₄₁H₃₆N₁₀O₁₁ [M+H]⁺ 845.26, found 845.26, *t*_{R} = 7.10 min.

### Compound 33:

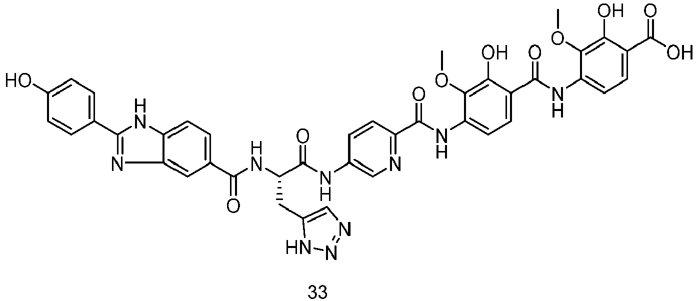

**¹H NMR** (DMSO-d₆ ,500MHz): δ 11.67 (s, 1H), 11.66 (br. s, 1H), 11.12 (s, 1H), 10.89 (s, 1H), 10.49 (s, 1H), 10.36 (s, 1H), 8.99 (d, *J* = 2.0 Hz, 1H), 8.98 (d, *J* =7.45 Hz, 1H), 8.36 (dd, *J* = 8.6, 2.1 Hz, 1H), 8.21 (d, *J* = 8.6 Hz, 1H), 8.17 (s, 1H), 8.12 (d, *J* = 9.3 Hz, 1H), 8.08 (d, *J* = 8.4 Hz, 2H), 8.06 (d, *J* = 8.4 Hz, 1H), 7.89-7.86 (m, 2H), 7.74 (br. s, 1H), 7.71 (d, *J* = 8.9 Hz, 1H), 7.60 (d, *J* = 8.9 Hz, 1H), 7.01 (d, *J* = 8.4 Hz, 2H), 4.98-4.97 (m, 1H), 3.92 (s, 3H), 3.88 (s, 3H), 3.34-3.31 (m, 1H), 3.28-3.26 (m, 1H). **(¹H,¹³C)-HSQC** (126 MHz, DMSO) δ 139.97, 127.77, 123.43, 110.76, 129.76, 110.82, 126.87, 126.17, 116.62, 54.92, 60.67, 61.27, 27.75; **HRMS (ESI):** m/z calculated for C₄₁H₃₄N₁₀O₁₁ [M+H]⁺ 843.24, found 843.24, *t*_{R} = 6.50 min.

### Test for biological activity

### Strains:

*E. coli* DSM 1116; S. *typhimurium* TA100; *Bacillus subtilis* DSM10; and *Micrococcus luteus* DSM1790

### Biological testing:

The tests were performed using the micro dilution method.

### Microdilution assay:

The determination of MIC values was performed according to the ninth edition of the Approved Standard M07-A9 (CLSI. Methods for Dilution Antimicrobial Susceptibility Tests for Bacteria That Grow Aerobically; Approved Standard-Ninth Edition. CLSI document M07-A9. Wayne, PA: Clinical and Laboratory Standards Institute; 2012.)

The test was carried out for four different bacterial strains (E.coli DSM 1116 [gram negative], B. subtilis DSM 10 [gram positive], M. luteus DSM 1790 [gram positive], S. typhimurium TA100 [gram negative]). 20 µL of cryo stock of each strain were inoculated in 20 mL of LB media (Lysogeny broth: 10 g/L peptone, 5 g/L yeast extract, 5 g/L NaCl) followed by incubation over night at 37°C, 200 rpm. The test inoculum was adjusted by the 0.5 McFarland Standard (OD625 from 0.08 to 0.1). Within 15 min of preparation, the adjusted inoculum suspension was diluted in MHBII media (BBL TM Mueller-Hinton Broth II, Becton, Dickinson and Company, New Jersey/USA) so that each well contained approximately 5 x 105 CFU/mL in a final volume of 100 µL. 95 µL of the inoculum were applied per well and 5 µL of the (diluted) antibiotic substance were added.

Previously the dry antibiotic compounds were dissolved in DMSO (100%) with a concentration of 2560 µg/mL and the resulting stock solutions were further diluted in DMSO (100%). 5 µL of each antibiotic dilution were applied to the microdilution tray to reach final concentrations of 64 µg/mL to 0.008 µg/mL. One row of each well plate was left as a growth control without antibiotic substances and another row of the microdilution tray was used as sterility control (only MHB II-media). The antimicrobial effect of the solvent (DMSO) was tested by adding 5 µL DMSO to several wells without antibiotics. Purity check and cell titer control were performed according to International Standard M07-A9 on Mueller-Hinton II Agar (Mueller Hinton II Broth, 15 g/L agar-agar). Both microdilution trays and agar plates were incubated at 37°C for 20 h and subsequently analyzed visually.

The results are summarized in table 1. The potency of an antibiotic is determined by the minimum inhibitory concentration (MIC). Contrary to intuition, a particularly low value is equated with a high potency.

**Table 1: Antibacterial activity of compounds according to the invention against selected strains**

| Compound | MIC in µg · mL⁻¹ | | | | | |
|---|---|---|---|---|---|---|
| | Gram-negative | | | Gram-positive | | |
| | *E. coli* DSM 1116 | *E. coli* BW25113 | S. typhimurium TA 100 | B. subtilis DSM 10 | *M. luteus* DSM 1790 | *M. phlei* DSM 750 |
| CIP | 0.016 | ≤ 0.016 | ≤ 0.016 | 0.125 | 1.0 | 0.25 |
| 0 | 0.016 | n.d. | ≤ 0.016 | 0.125 | 0.5 | 1.0 |
| 1 | ≤ 0.016 | 0.031 | ≤ 0.016 | 0.25 | 0.25 | 1.0 |
| 2a | 0.063 | 0.063 | ≤ 0.016 | 0.25 | 0.5 | 1.0 |
| 3 | 0.5 | 0.25 | 0.125 | 0.5 | 1.0 | 4.0 |
| 4 | 0.063 | 0.063 | ≤ 0.016 | 0.25 | 0.5 | 1.0 |
| 5 | 0.063 | 0.125 | 0.031 | 2.0 | 2.0 | 1.0 |
| 6 | 0.031 | 0.031 | ≤ 0.016 | 0.25 | 0.25 | 0.5 |
| 9 | 0.5 | 0.25 | 0.125 | 2.0 | 2.0 | 4.0 |
| 14 | 0.063 | 0.063 | 0.063 | 0.5 | 0.5 | 1.0 |
| 15 | 0.25 | 0.25 | 0.063 | 2.0 | 8.0 | 8.0 |
| 16 | 0.063 | 0.063 | 0.031 | 0.031 | 0.063 | 0.25 |
| 17 | 0.125 | 0.125 | 0.063 | 0.063 | 0.125 | 0.5 |
| 18 | 0.063 | 0.063 | 0.016 | 0.063 | 0.25 | 0.5 |
| 18b | 2.0 | 1.0 | 0.125 | 0.25 | 0.25 | 1.0 |
| 32 | 0.5 | 0.25 | 0.125 | 2 | 4 | 8 |
| 33 | ≥8 | ≥8 | ≥8 | ≥8 | ≥8 | ≥8 |

Table 1 also shows the MIC values of ciprofloxacin (CIP a fluoroquino-Ion), the gold standard of antibiotics, which has been tested in direct comparison. Furthermore, Compound 0 is a lead structure that has been optimized up to this point, and further modifications have been made to the template.

## Claims

1. A compound **characterized by** general formulae (I) 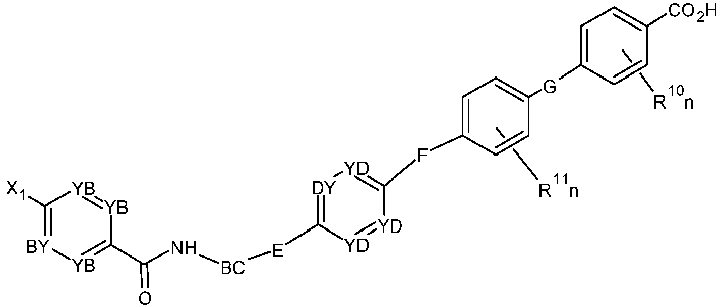
a) with X₁ in formulae being
a substituted or unsubstituted C₁-C₁₆ alkyl, in particular a substituted or unsubstituted C₁-C₈ alkyl;
a substituted or unsubstituted C₂-C₁₆ alkenyl, a substituted or unsubstituted C₂-C₈ alkenyl;
a substituted or unsubstituted C₂-C₁₆ alkynyl, a substituted or unsubstituted C₂-C₈ alkynyl;
-NHCNHRₐ;
-(NHCN)Rₐ, wherein (NHCN) forms a ring with the benzyl of formulae (Ia);
E-CONH-, wherein E is a substituted or unsubstituted C₅-C₁₀ heteroaryl, a substituted or unsubstituted C₆-C₁₀ aryl, or
-CHR_{b}-ORₐ;
with Rₐ being a substituted or unsubstituted C₆-C₁₀ aryl, a substituted or unsubstituted C₆-C₁₀ hetero aryl, and
R_{b} being H, C₁-C₆ alkyl, preferably C₁-C₄ alkyl;
b) with at least one, preferably at least two or all of E, F, G being -CO-NH-; and/or
with at least one of E, F and G being independently from each other selected from the group comprising 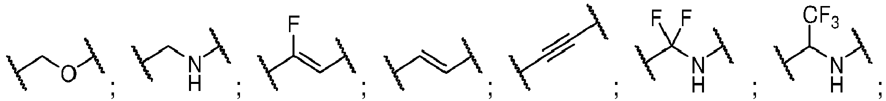 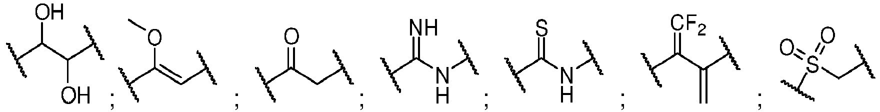 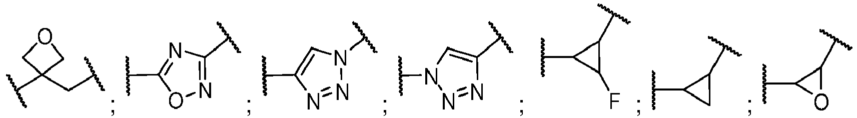 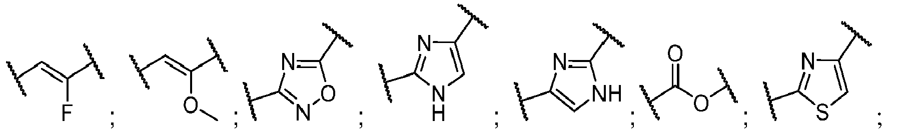 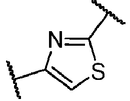 Or E, F, G are part of a ring formed with one of the adjacent phenyl rings, such as 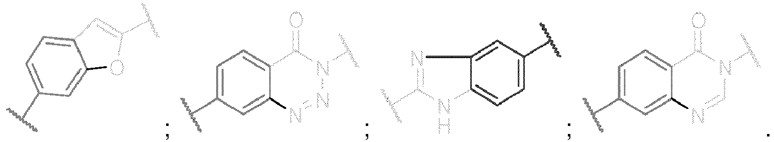
c) with BC being selected from 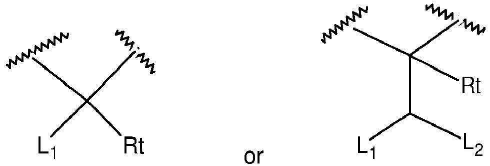
with L₁ being a substituted or unsubstituted aromatic heterocycle or a substituted or unsubstituted non-aromatic heterocycle, or -NHR^{d} or -NR^{d}₂;
with Rt being selected from H or C₁-C₄ alkyl,
with L₁ and Rt forming a non-aromatic heterocycle, in particular a N-heterocyclic ring, which is optionally substituted,
with L₂ being selected from -H, -OH, -OR^{d}, and substituted or unsubstituted -C₁-C₄ alkyl, C₁-C₆ alkoxycarbonyl and C₁-C₆ alkylaminocarbonyl,
with R^{d} being selected from a substituted or unsubstituted C₁-C₁₆ alkyl, , a substituted or unsubstituted C₂-C₁₆ alkenyl, in particular a substituted or unsubstituted C₁-C₈ alkyl, a substituted or unsubstituted C₂-C₈ alkenyl, a substituted or unsubstituted C₃-C₁₀ cycloalkyl, and all moieties optionally substituted with F,
or with BC being selected from 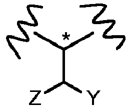
with Y being selected from -CN, -C(=O)OH, -C(=O)OCH₃, -C(=O)OCH₂CH₃, - C(=O)NHCH₃, -C(=O)NHCH₂CH₃, -C(=O)N(CH₃)₂, -C(=O)N(CH₂CH₃)₂, - C(=O)N(CH₃)(CH₂CH₃), -CF₃ or -C(=O)NH₂, and
with Z being selected from -H, -OH, -CH₃, -CH₂CH₃, -CCH, -OCH₃ ,-NH2, -NHCH₃, -N(CH₃)₂, -N(CH₃)₃⁺,
d) with n of R¹⁰ₙ and n of R¹¹ₙ being independently from each other 0, 1, 2, 3 or 4, in particular n of R¹⁰ₙ and n of R¹¹ₙ being 0, 1, 2 or 3, and
with each R¹⁰ and R¹¹ being selected independently from any other R¹⁰ and R¹¹ from -OH, -F, -Cl, -Br, -I, -CCH, -CN, -OC₁-C₆ alkyl, -OCF₃, -NH₂, -NHCH₃, -N(CH₃)₂, - C₁-C₆ alkyl, -OCH₂-NH₂, -OCH₂-CO₂H, -OCH₂-CN, in particular from -OH, -F, -OCH₃, - OC₂H₅, -OiC₃H₇, -OnC₃H₇, -OCF₃, more particular -OH, OCH₃, -OC₂H₅ or -OiPr respectively;
e) with YB being independently from each other N or CR¹⁴ and YD being independently from each other N or CR¹³;
with each R¹³ and R¹⁴ independently being selected from -H, -OH, -F, -OCH₃, - OC₂H₅, -OC₃H₇, -OCF₃, -CF₃ or -(CH₂)ₘ-ORₐ,
with Rₐ being selected from hydrogen, -CH₃, -CH₂CH₃, -CH₂CH₂CH₃, - CH₂CH₂CH₂CH₃, -CH(CH₃)₂, -CH₂CH(CH₃)₂, -C(CH₃)₃, -C₆H₅ -CH₂C₆H₅,
with m being selected from 1 or 2;
more particularly with R¹³ being -H, -OH, -OCH₃, -OC₂H₅ or -OiPr and R¹⁴ being -H or -F, respectively
wherein the following structure is disclaimed:

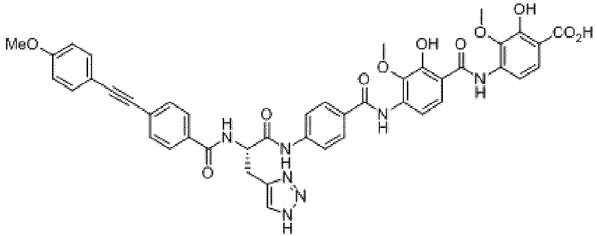

2. Compound according to claim 1, **characterized by** the general formula (1a) 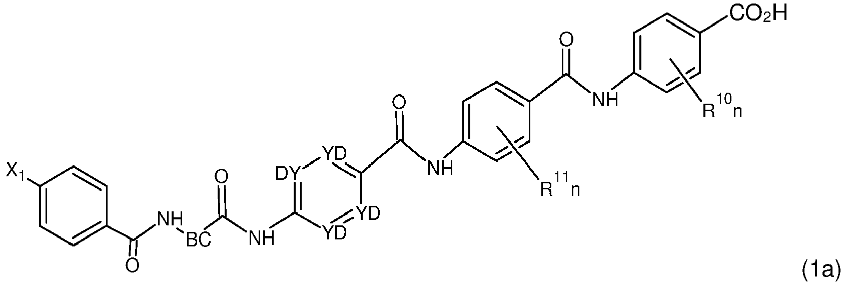
a) with X₁ in formulae (Ia) being
a substituted or unsubstituted C₁-C₁₆ alkyl, in particular a substituted or unsubstituted C₁-C₈ alkyl;
a substituted or unsubstituted C₂-C₁₆ alkenyl, a substituted or unsubstituted C₂-C₈ alkenyl;
a substituted or unsubstituted C₂-C₁₆ alkynyl, a substituted or unsubstituted C₂-C₈ alkynyl;
-NHCNHRₐ;
-(NHCN)Rₐ, wherein (NHCN) forms a ring with the benzyl of formulae (Ia);
- E-CONH-, wherein E is a substituted or unsubstituted C₅-C₁₀ heteroaryl, a substituted or unsubstituted C₆-C₁₀ aryl, or
-CHR_{b}-ORₐ;
with Rₐ being a substituted or unsubstituted C₆-C₁₀ aryl, a substituted or unsubstituted C₆-C₁₀ hetero aryl, and
R_{b} being H, C₁-C₆ alkyl, preferably C₁-C₄ alkyl;
c) with BC being selected from 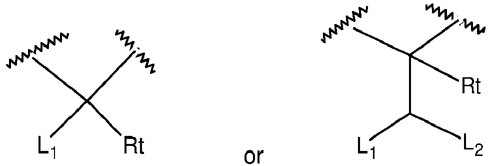
with L₁ being a substituted or unsubstituted aromatic heterocycle or a substituted or unsubstituted non-aromatic heterocycle, or -NHR^{d} or -NR^{d}₂;
with Rt being selected from H or C₁-C₄ alkyl,
with L₁ and Rt forming a non-aromatic heterocycle, in particular a N-heterocyclic ring, which is optionally substituted,
with L₂ being selected from -H, -OH, -OR^{d}, and substituted or unsubstituted -C₁-C₄ alkyl, C₁-C₆ alkoxycarbonyl and C₁-C₆ alkylaminocarbonyl,
with R^{d} being selected from a substituted or unsubstituted C₁-C₁₆ alkyl, , a substituted or unsubstituted C₂-C₁₆ alkenyl, in particular a substituted or unsubstituted C₁-C₈ alkyl, a substituted or unsubstituted C₂-C₈ alkenyl, a substituted or unsubstituted C₃-C₁₀ cycloalkyl, and all moieties optionally substituted with F,
or with BC being selected from 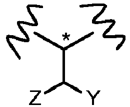
with Y being selected from -CN, -C(=O)OH, -C(=O)OCH₃, -C(=O)OCH₂CH₃, - C(=O)NHCH₃, -C(=O)NHCH₂CH₃, -C(=O)N(CH₃)₂, -C(=O)N(CH₂CH₃)₂, - C(=O)N(CH₃)(CH₂CH₃), -CF₃ or -C(=O)NH₂, and
with Z being selected from -H, -OH, -CH₃, -CH₂CH₃, -CCH, -OCH₃ ,-NH2, -NHCH₃, -N(CH₃)₂, -N(CH₃)₃⁺,
d) with n of R¹⁰ₙ and n of R¹¹ₙ being independently from each other 0, 1, 2, 3 or 4, in particular n of R¹⁰ₙ and n of R¹¹ₙ being 0, 1, 2 or 3, and
with each R¹⁰ and R¹¹ being selected independently from any other R¹⁰ and R¹¹ from -OH, -F, -Cl, -Br, -I, -CCH, -CN, -OC₁-C₆ alkyl, -OCF₃, -NH₂, -NHCH₃, -N(CH₃)₂, - C₁-C₆ alkyl, -OCH₂-NH₂, -OCH₂-CO₂H, -OCH₂-CN, in particular from -OH, -F, -OCH₃, - OC₂H₅, -OiC₃H₇, -OnC₃H₇, -OCF₃, more particulary -OH, OCH₃, -OC₂H₅ or -OiPr respectively;
e) with Y being independently from each other N or CH;
wherein the following structure is disclaimed:

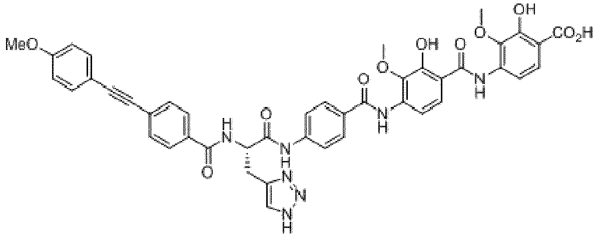

3. Compound according to claim 1 or 2, **characterized in that**
X₁ in formulae (Ia) is -CH₂CHRₐ, -CHCHRₐ, -CCRₐ,
wherein Rₐ being a substituted or unsubstituted C₆-C₁₀ aryl, substituted or unsubstituted C₅-C₁₀ heteroaryl, in particular N-heteroaryl, wherein at least one substituent may be -OH, -OAlkyl, in particular -OCH₃, -CN, halogen, in particular F, Rₐ being most preferably phenyl, methoxyphenyl, cyano benzyl, fluoro benzyl.

4. Compound according to claim 3, **characterized in that**
X₁ in formulae (Ia) is selected from -CC-C₆H₄-CN, - CC-C₆H₄-OH, - CC-C₆H₄-OCH₃, - CC-C₅H₃N-OCH₃, -CC-C₆H₅-F; - CHCH-C₆H₄-OH; - CH₂CH₂-C₆H₄-OH.

5. Compound according to claim 1 or 2 **characterized in that**
X₁ in formulae (Ia) is -NHCNHRₐ or -(NHCN)Rₐ, wherein (NHCN) forms a ring with the benzyl of formulae (Ia), wherein Rₐ is phenyl.

6. Compound according to claim 1 or 2, **characterized in that**
X₁ in formulae (Ia) is E-CONH-, wherein E being
a substituted or unsubstituted C₆-C₁₀ aryl, in particular substituted or unsubstituted benzyl, napthyl, wherein the substituent may be an alkyl, alkenyl or alkynyl, such as a C₁-C₆ alkyl, a C₂-C₈ alkenyl or a C₂-C₈ alkynyl,a substituted or unsubstituted N-heteroaryl, such as a triazoles or a tetrazole, imidazole, indole, benzimidazole, chinolin, or
-CHR_{b}-ORₐ, wherein Rₐ is phenyl and R_{b} is H or -CH₃.

7. Compound according to claim 1 **characterized by** general formula (1b) 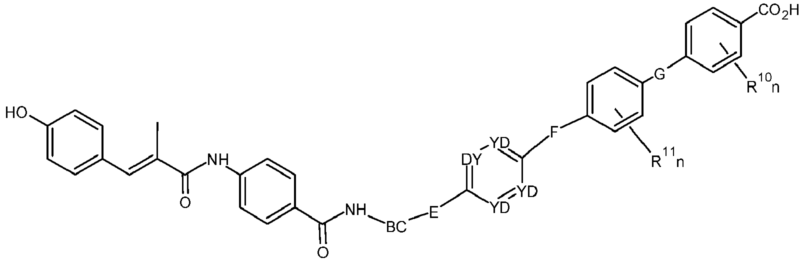 with at least one, preferably at least two of E, F, G being -CO-NH-; and
with at least one of E, F and G being independently from each other selected from the group comprising 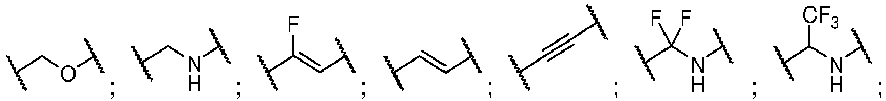 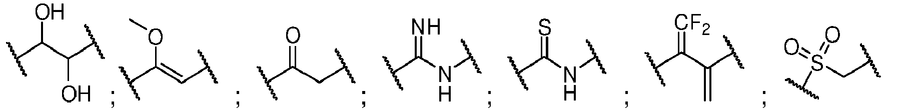 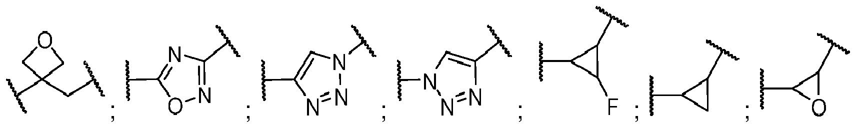 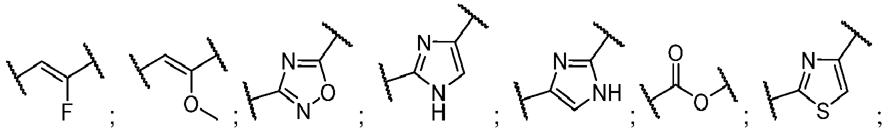 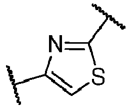 Or E, F, G are part of a ring formed with one of the adjacent phenyl rings, such as 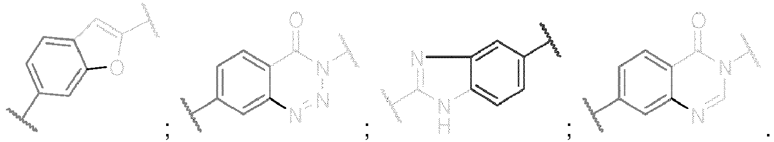
c) with BC being selected from 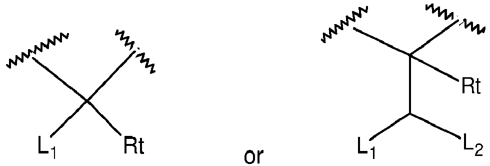
with L₁ being a substituted or unsubstituted aromatic heterocycle or a substituted or unsubstituted non-aromatic heterocycle, or -NHR^{d} or -NR^{d}₂;
with Rt being selected from H or C₁-C₄ alkyl,
with L₁ and Rt forming a non-aromatic heterocycle, in particular a N-heterocyclic ring, which is optionally substituted,
with L₂ being selected from -H, -OH, -OR^{d}, and substituted or unsubstituted -C₁-C₄ alkyl, C₁-C₆ alkoxycarbonyl and C₁-C₆ alkylaminocarbonyl,
with R^{d} being selected from a substituted or unsubstituted C₁-C₁₆ alkyl, , a substituted or unsubstituted C₂-C₁₆ alkenyl, in particular a substituted or unsubstituted C₁-C₈ alkyl, a substituted or unsubstituted C₂-C₈ alkenyl, a substituted or unsubstituted C₃-C₁₀ cycloalkyl, and all moieties optionally substituted with F,
or with BC being selected from 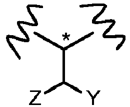
with Y being selected from -CN, -C(=O)OH, -C(=O)OCH₃, -C(=O)OCH₂CH₃, - C(=O)NHCH₃, -C(=O)NHCH₂CH₃, -C(=O)N(CH₃)₂, -C(=O)N(CH₂CH₃)₂, - C(=O)N(CH₃)(CH₂CH₃), -CF₃ or -C(=O)NH₂, and
with Z being selected from -H, -OH, -CH₃, -CH₂CH₃, -CCH, -OCH₃ ,-NH2, -NHCH₃, -N(CH₃)₂, -N(CH₃)₃⁺,
d) with n of R¹⁰ₙ and n of R¹¹ₙ being independently from each other 0, 1, 2, 3 or 4, in particular n of R¹⁰ₙ and n of R¹¹ₙ being 0, 1, 2 or 3, and
with each R¹⁰ and R¹¹ being selected independently from any other R¹⁰ and R¹¹ from -OH, -F, -Cl, -Br, -I, -CCH, -CN, -OC₁-C₆ alkyl, -OCF₃, -NH₂, -NHCH₃, -N(CH₃)₂, - C₁-C₆ alkyl, -OCH₂-NH₂, -OCH₂-CO₂H, -OCH₂-CN, in particular from -OH, -F, -OCH₃, - OC₂H₅, -OiC₃H₇, -OnC₃H₇, -OCF₃, more particulary -OH, OCH₃, -OC₂H₅ or -OiPr respectively;
e) with Y being independently from each other N or CH;

8. Compound according to claim 7, **characterized in that** one of E, F, G are as follows:
E is 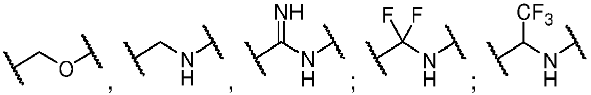
F is 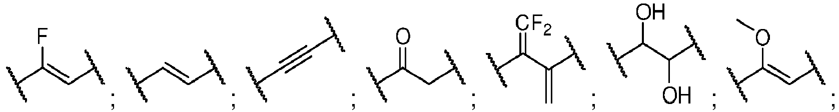 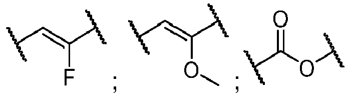
G is 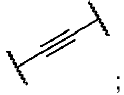
wherein at least one, preferably two of E, F and G is -CO-NH-.

9. Compound according to one of the preceding claims, **characterized in that** moiety L₁ is a five membered or six membered aromatic heterocycle or 3-7 membered non-aromatic heterocycle, preferably a five membered or six membered aromatic N-heterocycle or non-aromatic N heterocycle that may be substituted or unsubstituted.

10. Compound according to one of the preceding claims, **characterized in that** L₁ is a five membered aromatic N-heterocycle selected from a group comprising substituted or unsubstituted
- pyrroles, imidazoles, pyrazoles, triazoles, tetrazoles;
- pyrazolone, preferably 3H-pyrazol-3-ones, 4H-pyrazol-4-ones, 1,2-dihydro-3H-pyrazol-3-ones, 2,4-dihydro-3H-pyrazol-3-ones, triazolones, preferably 1,2,4-triazol-3-one, imidazolones, pyrrolidones,
- thiadiazoles, preferably 1,3,4-thiadiazoles, thiazoles, isothiazoles, thiazolidinediones; and
- isoxazoles, oxazoles, oxadiazoles (1,3,4-oxadiazoles, 1,2,4-oxadiazoles).

11. Compound according to one of the preceding claims, **characterized in that** L₂ is selected from -H, -OH, -OR^{d}, and -CH₃, -C₂H₆ or -C₃H₇, with R^{d} being substituted or unsubstituted C₁-C₈ alkyl, preferably a C₁-C₃ alkyl.

12. Compound according to one of the preceding claims, **characterized in that** Z being H and Y being CN or -C(=O)NH₂, more preferably Z being H and Y being CN.

13. Compound according to one of the preceding claims, **characterized in that** n of R¹⁰ₙ and n of R¹¹ₙ being 0, 1, 2, 3 or 4, in particular n of R¹⁰ₙ and n of R¹¹ₙ being 0, 1, 2 or 3, and with each R¹⁰ and with each R¹¹ independently from any other R¹⁰ being selected from -OH, -F, -OCH₃, -OC₂H₅, -OC₃H₇, -OCF₃, -CF₃ or -(CH₂)m-ORc,
with Rc being selected from hydrogen, -CH₃, -CH₂CH₃, -CH₂CH₂CH₃, - CH₂CH₂CH₂CH₃, -CH(CH₃)₂, -CH₂CH(CH₃)₂, -C(CH₃)₃, -C₆H₅ -CH₂C₆H₅,
with m being selected from 1 or 2.
more particularly with one R¹⁰ or R¹¹ being -OH and the other R¹⁰ or R¹¹ being - OCH₃, -OC₂H₅ or -OiPr respectively.

14. Compound according to one of the preceding claims for use in a method of treatment of diseases, in particular for use in a method of treatment of bacterial infections by gram-negative or gram-positive bacterial strains.

15. Compound for use in a method according to claim 14, wherein the bacterial infection is an infection by one of the genus Acinetobacter, Bordatella, Borellia, Brucella, Camphylobacter, Chlamydia, Chlamydophila, Enterobacter, Escherichia, Francisella, Haemophilus, Helicobacter, Klebisella, Legionella, Leptospira, Morganella Moraxella, Neisseria, Proteus, Pseudomonas, Rickettsia, Shigella, Salmonella, Stenotrophomonas, Treponema or Yersinia.
